# EUROPÄISCHE PATENTSCHRIFT

(11) **EP 1 507 564 B1**
(45) Veröffentlichungstag und Bekanntmachung des Hinweises auf die Patenterteilung: **10.08.2016**
(21) Anmeldenummer: 03720516.8
(22) Anmeldetag: 23.04.2003
(51) Int. Cl.: A61L 15/24, C08K 3/24

(54) **WASSERABSORBIERENDE, DIE ZERSETZUNG VON KÖRPERFLÜSSIGKEITEN VERZÖGERNDE POLYMERTEILCHEN, DIESE BEINHALTENDE VERBUNDE SOWIE DEREN VERWENDUNG**
WATER-ABSORBING POLYMER PARTICLES INHIBITING THE BREAKDOWN OF BODY FLUIDS, COMPOSITES COMPRISING THE SAME AND USE THEREOF
PARTICULES POLYMERES HYDROABSORBANTES RETARDANT LA DECOMPOSITION DE FLUIDES CORPORELS, COMPOSITES CONTENANT CES PARTICULES ET UTILISATION DE CES PARTICULES ET COMPOSITES

(30) Priorität: 23.04.2002 DE 10218147
(43) Veröffentlichungstag der Anmeldung: 23.02.2005
(73) Patentinhaber: Evonik Degussa GmbH, 45128 Essen (DE)
(72) Erfinder: HARREN, Jörg, 47807 Krefeld (DE); ISSBERNER, Jörg, 47877 Willich-Neersen (DE); SCHMIDT, Harald, 47918 Tönisvorst (DE)
(74) Vertreter: Lang, Arne
(86) Internationale Anmeldenummer: PCT/EP2003/004203
(87) Internationale Veröffentlichungsnummer: WO 2003/090799

(56) Entgegenhaltungen:
- EP-A- 0 255 248
- WO-A-01/41819

## Beschreibung

Die vorliegende Erfindung betrifft wasserabsorbierende, die Zersetzung von Körperflüssigkeiten verzögernde bzw. antimikrobielle Polymerteilchen; Verfahren zur Herstellung solcher Polymerteilchen; die durch diese Verfahren erhältlichen Polymerteilchen, Fasern, Folien, Schäume oder Formmassen, beinhaltend solche Polymerteilchen; Verbunde, beinhaltend solche Polymerteilchen; sowie die Verwendung solcher wasserabsorbierender Polymerteilchen, Verbund, Fasern, Folien, Schäume oder Formmassen zur Herstellung von Hygiene- und Medizinartikeln wie Windeln, Damenbinden oder Inkontinenzartikeln und Wundauflagen zur Vorbeugung oder Behandlung von durch Ausscheidungen und Körperflüssigkeiten hervorgerufenen Hautirritationen, vorzugsweise Windeldermatitis, sowie zur Behandlung offener Wunden.

Durch den beim Tragen von Hygieneartikeln auftretenden Kontakt der Haut des Trägers mit Ausscheidungen und Körperflüssigkeiten wie Urin und Kot des Trägers können an den durch den Hygieneartikel abgedeckten Körperteilen des Trägers Hautirritationen, insbesondere Entzündungen, beispielsweise die sogenannte Windeldermatitis auftreten. Das Auftreten solcher Hautirritationen hat meist zur Folge, dass die Hygieneartikel bis zur Abheilung der Hautirritation nicht mehr getragen werden können. Für den Träger ist neben den mit der Hautirritation auftretenden Schmerzen dadurch ein erheblicher Verlust an Beweglichkeit und Unabhängigkeit verbunden.

Nach allgemein herrschender Meinung werden derartige Hautirritationen vorwiegend durch im Urin oder Kot des Trägers des Hygieneartikels enthaltene Reizstoffe, insbesondere durch deren Zersetzung hervorgerufen. Diese kann durch Mikroben hervorgerufen oder beschleunigt werden.

Weiterhin entstehen neben dem Eigengeruch von Urin und Kot über die Zeit weitere unangenehme Gerüche, die vor allem auf deren Zersetzung zurückzuführen sind. Derartige Gerüche werden von den Trägern und seiner Umwelt meist als äußerst unangenehm empfunden.

In der Vergangenheit wurden zahlreiche Versuche unternommen, wasserabsorbierende Polymere zur Verfügung zu stellen, die die Zersetzung von Körperflüssigkeiten verzögernde Wirkungen zeigen oder zur Unterdrückung der Bildung unangenehmer Gerüche beitragen.

U.S. 6,277,772 B1 lehrt superabsorbierende Polymerpulver mit einer Größe im Bereich von 100 bis 800 µm mit einem Zeolithpulver zu beaufschlagen, das eine Teilchengröße im Bereich zwischen 0,5 und 20 µm besitzt und mit antimikrobiellen Kationen, beispielsweise Silberionen, beladen wurde. Die in diesem U.S.-Patent offenbarte Lehre ist nachteilhaft, da zum einen das antimikrobielle wasserabsorbierende Polymer in mehreren Schritten hergestellt werden muss, und andererseits liegt ein Nachteil darin, dass bei dem Umgang und bei der Beaufschlagung mit Zeolithen der vorstehend genannten Teilchengröße die Gefahr von Staubbildung erhöht ist, die gesundheitlichen Gefahren sowohl für die mit der Herstellung eines solchen antimikrobiellen wasserabsorbierenden Polymers betrauten Arbeitskräfte als auch bei den Trägern dieser antimikrobiellen wasserabsorbierenden Polymere beinhaltenden Hygieneartikeln erhöhen.

WO 01/41819 A1 lehrt die Verwendung von schwerlöslichen Silbersalzen oder kolloidalem Silber bei der Herstellung von antimikrobiellen wasserabsorbierenden Polymeren. So können gemäß dieser WO-Schrift kolloidales Silber oder die unlöslichen, bzw. schwerlöslichen Silbersalze der Monomerlösung vor der Polymerisation zugesetzt werden oder das kolloidale Silber oder die unlöslichen, bzw. die schwerlöslichen Silbersalze auf die bereits getrockneten wasserabsorbierenden Polymerpartikel aufgebracht werden. Beide Verfahrensvarianten sind nachteilhaft. Bei der Zugabe von kolloidalem Silber oder unlöslichen, bzw. schwerlöslichen Silbersalzen, zur Monomerlösung wird aufgrund der Schwer- bzw. Unlöslichkeit eine vergleichsweise inhomogene Verteilung der Silbersalze oder des kolloidalen Silbers erreicht. Das oberflächliche Aufbringen von schwer- bzw. unlöslichen Silbersalzen oder kolloidalem Silber führt dazu, dass diese vergleichsweise schlecht an der Oberfläche des wasserabsorbierenden Polymerteilchens anhaften und meist weitere Hilfsstoffe, beispielsweise Zeolithe oder Tenside, zur Fixierung der schwer- bzw. unlöslichen Silbersalze oder des kolloidalen Silbers auf der Oberfläche des wasserabsorbierenden Polymerteilchens notwendig sind. Sowohl die inhomogene Verteilung als auch die schlechte oberflächliche Anhaftung führen außerdem zu einer erhöhten Separierung von Silber bzw. Silbersalzen und den wasserabsorbierenden Polymerteilchen während Transport, Lagerung und insbesondere der hohen mechanische Belastung erzeugenden Konvertierung.

Allgemein bekannt ist auch die durch lang andauernde Exposition von Silber mit Haut auftretende Agyose. Die Absorption von Silber führt ferner leicht zu permanenten Hautverfärbungen. Hieraus ergibt sich, dass auch hier eine oberflächliche und inhomogene Verteilung mit Bereichen deutlich erhöhter Konzentration von Silbersalzen oder kolloidalen Silbers nachteilig ist, da diese zu einer hohen Exposition mit der Hautoberfläche führt und damit das Risiko einer Agyose erhöht.

Allgemein liegt die Aufgabe der vorliegenden Erfindung darin, die sich aus dem Stand der Technik ergebenden Nachteile zu überwinden.

Insbesondere besteht eine erfindungsgemäße Aufgabe darin, wasserabsorbierende antimikrobielle Polymerteilchen zur Verfügung zur stellen, die über ausreichende antimikrobielle Wirkungen verfügen, die über einen ausreichend langen Zeitraum anhalten.

Ferner besteht eine erfindungsgemäße Aufgabe darin, eine ausreichende antimikrobielle Wirkung mit einer möglichst geringfügigen Absenkung des pH-Wertes unter den normalen pH-Wert der gesunden Hautoberfläche zu erreichen.

Weiterhin liegt eine erfindungsgemäße Aufgabe darin, ein Verfahren zur Verfügung zu stellen, mit dem antimikrobielle wasserabsorbierende Polymerteilchen in möglichst wenigen und kostengünstigen Verfahrensschritten hergestellt werden.

Zudem besteht eine erfindungsgemäße Aufgabe darin, antimikrobielle wasserabsorbierende Polymerteilchen zur Verfügung zu stellen deren Fähigkeit zur Zersetzung von Körperflüssigkeiten nicht durch Transport, Lagerung oder Konvertierung wesentlich beeinträchtig wird.

Gemäß einer weiteren erfindungsgemäßen Aufgabe sollen antimikrobielle wasserabsorbierende Polymerteilchen zur Verfügung gestellt werden, die in Hygieneartikel eingearbeitet werden können, die zur Vorbeugung bzw. Behandlung von Hautirritationen, insbesondere von Windeldermatitis bei, vorzugsweise bei verminderten Agyose-Risiko, geeignet sind.

Ferner besteht eine andere erfindungsgemäße Aufgabe darin, ein Verfahren zu schaffen, dass es erlaubt, antimikrobielle wasserabsorbierende Polymerteilchen herzustellen, in denen das antimikrobielle Mittel möglichst gleichmäßig verteilt ist.

Die vorstehenden Aufgaben werden gelöst durch ein wasserabsorbierendes, antimikrobielles Polymerteilchen beinhaltend
- 1 bis 500 ppm, bevorzugt 5 bis 300 ppm, besonders bevorzugt 10 bis 70 ppm und darüber hinaus bevorzugt 20 bis 50 ppm eines auf einem Silbersalz basierenden Silberions als Ionenkonzentration, Silbersalz AgₘXₙ ein Löslichkeitsprodukt, vorzugsweise ein Löslichkeitsprodukt in Wasser, besonders bevorzugt ein Löslichkeitsprodukt in Wasser bei einer Temperatur von 25°C, von mindestens 1 × 10⁻⁸ (Mol/l)^{m+n}, vorzugsweise von mindestens 1 × 10⁻⁷ (Mol/l)^{m+n}, besonders bevorzugt von mindestens 1 × 10⁻⁶ (Mol/l)^{m+n}, darüber hinaus bevorzugt von mindestens 1 × 10⁻⁵ (Mol//)^{m+n}, darüber hinaus noch mehr bevorzugt von mindestens 1 × 10⁻⁴ (Mol/l)^{m+n} und am meisten bevorzugt von mindestens 1 × 10⁻³ (Mol/l)^{m+n} besitzt;
- mindestens 10 Gew.-%, vorzugsweise mindestens 50 Gew.-% und besonders bevorzugt mindestens 80 Gew.-%, bezogen auf das Polymerteilchen, eines wasserabsorbierenden Polymers basierend auf:
   (α1) 50 bis 99,99 Gew.-%, vorzugsweise 70 bis 99,99 Gew.-% und besonders bevorzugt 85 bis 99,99 Gew.-% polymerisierten, ethylenisch ungesättigten, säuregruppenhaltigen Monomeren oder deren Salze oder deren Mischungen,
   (α2) 0 bis 40 Gew.-%, bevorzugt 1 bis 30 Gew.-% und besonders bevorzugt 5 bis 20 Gew.-% polymerisierten, monoethylenisch ungesättigten, mit (α1) polymerisierbaren Monomeren,
   (α3) 0,01 bis 5 Gew.-%, vorzugsweise 0,1 bis 3 Gew.-% und besonders bevorzugt 0,5 bis 2 Gew.-% eines oder mehrerer Vernetzer, wobei diese Vernetzer Verbindungen sind, die mindestens zwei ethylenisch ungesättigte Gruppen innerhalb eines Moleküls aufweisen (Vernetzerklasse I),Verbindungen, die mindestens zwei funktionelle Gruppen aufweist, die mit funktionellen Gruppen der Monomeren (α1) oder (α2) in einer Kondensationsreaktion, in einer Additionsreaktion oder in einer Ringöffnungsreaktion reagieren können (Vernetzerklasse II) Verbindungen, die mindestens eine ethylenisch ungesättigte Gruppe und mindestens eine funktionelle Gruppe, die mit funktionellen Gruppen der Monomeren (α1) oder (α2) in einer Kondensationsreaktion, in einer Additionsreaktion oder in einer Ringöffnungs-reaktion reagieren kann (Vernetzerklasse III), aufweisen, oder polyvalente Metallkationen (Vernetzerklasse IV),
   (α4) 0 bis 30 Gew.-%, bevorzugt 1 bis 20 Gew.-% und besonders bevorzugt 5 bis 10 Gew.-% eines wasserlöslichen Polymeren sowie
   (α5) 0 bis 20 Gew.-%, bevorzugt 0,01 bis 7 Gew.-% und besonders bevorzugt 0,05 bis 5 Gew.-% eines oder mehrerer Hilfsstoffe, wobei als Hilfsstoffe, Stellmittel, Geruchsbinder, oberflächenaktive Mittel oder Antioxidantien eingesetzt werden, wobei die Summe der Gewichtsmengen (α1) bis (α5) 100 Gew.-% beträgt;
- gegebenenfalls geeignete Zusatzstoffe, die zur Stabilisierung des Silberions oder des Silbersalzes beitragen und Stoffe die durch Silber bedingte Verfärbung unterbinden oder zumindest aufhalten;
wobei die Konzentration des auf einem Silbersalz basierendes Silberions in höchstens 90 Vol.-% bevorzugt in höchstens 70 Vol.-%, besonders bevorzugt in höchstens 30 Vol.-% und darüber hinaus bevorzugt in keinem Bereich des wasserabsorbierenden, antimikrobiellen Polymerteilchens kleiner als 0,01 ppm, bevorzugt 0,5 ppm und besonders bevorzugt 0,9 ppm ist und wobei
X in der Formel Silbersalz AgₘXₙfür ein Anion aus der Gruppe von Nitrat, Carbonat, Sulfat, Hydrogensulfat, Alaun, Phosphat, Acrylat, Lactat, Acetat, Sulfonat, Benzoat, Triflourmethansulfonat oder Acrylat und wobei das wasserabsorbierende, antimikrobielle Polymerteilchen mit einem Nachvernetzer, von 0,01 bis 30 Gew.-%, bevorzugt 0,1 bis 20 Gew.-% und besonders bevorzugt 0,5 bis 10 Gew.-%, jeweils bezogen auf das Gewicht des unbehandelten wobei dieser Nachvernetzer aus der Gruppe von Diethylenglykol, Triethylenglykol, Polyethylenglykol, Glyzerin, Polyglyzerin, Propylenglykol, Diethanolamin, Triethanolamin, Polyoxypropylen, Oxyethylen-Oxypropylen-Blockcopolymere, Sorbitanfettsäureester, Polyoxyethylen-sorbitanfettsäureester, Trimethylolpropan, Pentaerytrit, Polyvinylalkohol, Sorbitol, 1,3-Dioxolan-2-on (Ethylencarbonat), 4-Methyl-1,3-dioxolan-2-on (Propylencarbonat), 4,5-Dimethyl-1,3-dioxolan-2-on, 4,4-Dimethyl-1,3-dioxolan-2-on, 4-Ethyl-1,3-dioxolan-2-on, 4-Hydroxymethyl-1,3-dioxolan-2-on, 1,3-Dioxan-2-on, 4-Methyl-1,3-dioxan-2-on, 4,6-Dimethyl-1,3-dioxan-2-on, 1,3-Dioxolan-2-on, Poly-1,3-dioxolan-2-on aufweist, ist, in einer Menge in einem Bereich von 0,01 bis 30 Gew.-%, bezogen auf das Gewicht des Polymerteilchens, nachvernetzt ist.

Die vorstehenden Aufgaben werden auch gelöst durch ein wasserabsorbierendes, antimikrobielles Polymerteilchen, wie vorstehend bereits beschrieben, beinhaltend
- 1 bis 500 ppm, bevorzugt 5 bis 300 ppm, besonders bevorzugt 10 bis 70 ppm und darüber hinaus bevorzugt 20 bis 50 ppm, bezogen auf das Polymerteilchen, eines auf einem Silbersalz ein Löslichkeitsprodukt, vorzugsweise ein Löslichkeitsprodukt in Wasser, besonders bevorzugt ein Löslichkeitsprodukt in Wasser bei einer Temperatur von 25°C, von mindestens 1 × 10⁻⁸ (Mol/l)^{m+n}, vorzugsweise von mindestens 1 × 10⁻⁷ (Mol/l)^{m+n}, besonders bevorzugt von mindestens 1 × 10⁻⁶ (Mol/l)^{m+n}, darüber hinaus bevorzugt von mindestens 1 × 10⁻⁵ (Mol/l)^{m+n}, darüber hinaus noch mehr bevorzugt von mindestens 1 × 10⁻⁴ (Mol/l)^{m+n} und am meisten bevorzugt von mindestens 1 × 10⁻³ (Mol/l)^{m+n} besitzt;
- mindestens 10 Gew.-%, vorzugsweise mindestens 50 Gew.-% und besonders bevorzugt mindestens 80 Gew.-%, bezogen auf das Polymerteilchen, eines wasserabsorbierenden Polymers basierend auf:
   (α1) 50 bis 99,99 Gew.-%, vorzugsweise 70 bis 99,99 Gew.-% und besonders bevorzugt 85 bis 99,99 Gew.-% polymerisierten, ethylenisch ungesättigten, säuregruppenhaltigen Monomeren oder deren Salze oder deren Mischungen,
   (α2) 0 bis 40 Gew.-%, bevorzugt 1 bis 30 Gew.-% und besonders bevorzugt 5 bis 20 Gew.-% polymerisierten, monoethylenisch ungesättigten, mit (α1) polymerisierbaren Monomeren,
   (α3) 0,01 bis 5 Gew.-%, vorzugsweise 0,1 bis 3 Gew.-% und besonders bevorzugt 0,5 bis 2 Gew.-% eines oder mehrerer Vernetzer,
   (α4) 0 bis 30 Gew.-%, bevorzugt 1 bis 20 Gew.-% und besonders bevorzugt 5 bis 10 Gew.-% eines wasserlöslichen Polymeren sowie
   (α5) 0 bis 20 Gew.-%, bevorzugt 0,01 bis 7 Gew.-% und besonders bevorzugt 0,05 bis 5 Gew.-% eines oder mehrerer Hilfsstoffe,
wobei die Summe der Gewichtsmengen (α1) bis (α5) 100 Gew.-% beträgt;
- gegebenenfalls geeignete Zusatzstoffe;
wobei die Konzentration des auf einem Silbersalz basierendes Silberions in höchstens 90 Vol.-%, bevorzugt in höchstens 70 Vol.-%, besonders bevorzugt in höchstens 30 Vol.-% und darüber hinaus bevorzugt in keinem Bereich des wasserabsorbierenden, antimikrobiellen Polymerteilchens kleiner als 0,01 ppm, bevorzugt 0,5 ppm und besonders bevorzugt 0,9 ppm ist und wobei
der pH-Wert des wasserabsorbierenden Polymers, vorzugsweise der pH-Wert von 1 g des Polymers in 1 1 Wasser gemäß ERT 400.1-99, in einem Bereich von 4,5 bis 7, bevorzugt von 5,2 bis 6,5 und besonders bevorzugt in einem Bereich von 5,3 bis 6,2 liegt.

In einer erfindungsgemäß bevorzugten Ausführungsform der erfindungsgemäßen wasserabsorbierenden, antimikrobiellen Polymerteilchen liegt die Konzentration des auf einem Silbersalz basierenden Silberions in keinem Bereich der wasserabsorbierenden, antimikrobiellen Polymerteilchen außerhalb der Ionenkonzentration +/- 30 %, bevorzugt +/- 15 % und besonders bevorzugt +/- 5 %, bezogen auf die Ionenkonzentration.

In einer erfindungsgemäß bevorzugten Ausführungsform der wasserabsorbierenden, antimikrobiellen Polymerteilchen weisen diese 1 bis 500 ppm, bevorzugt 5 bis 300 ppm, besonders bevorzugt 10 bis 70 ppm und darüber hinaus bevorzugt 20 bis 50 ppm, bezogen auf die Polymerteilchen, eines auf dem Silbersalz basierendes Silberions auf, wobei jeder Bereich der Polymerteilchen die vorstehend beschriebene Konzentration des Silberions aufweist.

Die erfindungsgemäßen wasserabsorbierenden antimikrobiellen Polymerteilchen sind in der Regel im wesentlichen kugelförmig, vorzugsweise wie durch einen Mahlprozess erhältlich, flockenartig oder liegen als Pellets vor. Vorzugsweise besitzen die erfindungsgemäßen wasserabsorbierenden antimikrobiellen Polymerteilchen eine Teilchengröße, die bei mindestens 20 Gew.-%, bevorzugt mindestens 50 Gew.-% und besonders bevorzugt mindestens 80 Gew.-% zwischen 150 und 850 µm liegt. Die Teilchengröße wird dadurch bestimmt, dass die Teilchen durch ein Sieb mit einer Maschenweite von 850 µm durchfallen und auf einem Sieb mit einer Maschenweite von 150 µm liegen bleiben.

Die monoethylenisch ungesättigten, säuregruppenhaltigen Monomere (α1) können teilweise oder vollständig, bevorzugt teilweise neutralisiert sein. Vorzugsweise sind die monoethylenisch ungesättigten, säuregruppenhaltigen Monomere zu mindestens 25 Mol-%, besonders bevorzugt zu mindestens 40 Mol-% und darüber hinaus bevorzugt zu 40-90 Mol-% neutralisiert. Die Neutralisation der Monomere (α1) kann vor oder auch nach der Polymerisation erfolgen. Ferner kann die Neutralisation mit Alkalimetallhydroxiden, Erdalkalimetallhydroxiden, Ammoniak sowie Carbonaten und Bicarbonaten erfolgen. Daneben ist jede weitere Base denkbar, die mit der Säure ein wasserlösliches Salz bildet. Auch eine Mischneutralisation mit verschiedenen Basen ist denkbar. Bevorzugt ist die Neutralisation mit Ammoniak oder mit Alkalimetallhydroxiden, besonders bevorzugt mit Natriumhydroxid oder mit Ammoniak.

Bevorzugte monoethylenisch ungesättigte, säuregruppenhaltige Monomere (α1) sind Acrylsäure, Methacrylsäure, Ethacrylsäure, α-Chloracrylsäure, α-Cyanoacrylsäure, β-Methylacrylsäure (Crotonsäure), a-Phenylacrylsäure, β-Acryloxypropionsäure, Sorbinsäure, α-Chlorsorbinsäure, 2'-Methylisocrotonsäure, Zimtsäure, p-Chlorzimtsäure, β-Stearylsäure, Itaconsäure, Citraconsäure, Mesaconsäure, Glutaconsäure, Aconitsäure, Maleinsäure, Fumarsäure, Tricarboxyethylen und Maleinsäureanhydrid, wobei Acrylsäure sowie Methacrylsäure besonders und Acrylsäure darüber hinaus bevorzugt sind.

Neben diesen carboxylatgruppenhaltigen Monomeren sind als monoethylenisch ungesättigte, säuregruppenhaltige Monomere (α1) des Weiteren ethylenisch ungesättigte Sulfonsäuremonomere oder ethylenisch ungesättigte Phosphonsäuremonomere bevorzugt.

Als ethylenisch ungesättigte Sulfonsäuremonomere sind Allylsulfonsäure oder aliphatische oder aromatische Vinylsulfonsäuren oder acrylische oder methacrylische Sulfonsäuren bevorzugt. Als aliphatische oder aromatische Vinylsulfonsäuren sind Vinylsulfonsäure, 4-Vinylbenzylsulfonsäure, Vinyltoluolsulfonsäure und Stryrolsulfonsäure bevorzugt. Als Acryl- bzw. Methacrylsulfonsäuren sind Sulfoethyl(meth)acrylat, Sulfopropyl(meth)acrylat, 2-Hydroxy-3-methacryloxypropylsulfonsäure sowie (Meth)Acrylamidoalkylsulfonsäuren wie 2-Acrylamido-2-methylpropansulfonsäure bevorzugt.

Als ethylenisch ungesättigte Phosphonsäuremonomere sind Vinylphosphonsäure, Allylphosphonsäure, Vinylbenzylphosponsäure, (Meth)acrylamidoalkylphosphonsäuren, Acrylamidoalkyldiphosphonsäuren, phosponomethylierte Vinylamine und (Meth)acrylphosphonsäurederivate bevorzugt.

Es ist erfindungsgemäß bevorzugt, dass das Polymer zu mindestens 50 Gew.-%, vorzugsweise zu mindestens 70 Gew.-% und darüber hinaus bevorzugt zu mindestens 90 Gew.-% auf carboxylatgruppenhaltigen Monomeren besteht. Es ist erfindungsgemäß besonders bevorzugt, dass das Polymer zu mindestens 50 Gew.-%, vorzugsweise zu mindestens 70 Gew.-% aus Acrylsäure besteht, die vorzugsweise zu mindestens 20 Mol-%, besonders bevorzugt zu mindestens 40 Mol-% neutralisiert ist.

Als ethylenisch ungesättigte, einen protonierten Stickstoff enthaltende Monomere, die neben den monoethylenisch ungesättigtem, säuregruppenhaltigen Monomeren (α1) vorliegen können, sind vorzugsweise Dialkylaminoalkyl(meth)acrylate in protonierter Form, beispielsweise Dimethylaminoethyl(meth)acrylat-Hydrochlorid oder Dimethylaminoethyl(meth)acrylat-Hydrosulfat, sowie Dialkylaminoalkyl-(meth)acrylamide in protonierter Form, beispielsweise Dimethylaminoethyl(meth)acrylamid-Hydrochlorid, Diemethylaminopropyl(meth)acrylamid-Hydrochlorid, Diemethylaminopropyl(meth)acrylamid-Hydrosulfat oder Dimethylaminoethyl(meth)acrylamid-Hydrosulfat bevorzugt.

Als ethylenisch ungesättigte, einen quarternierten Stickstoff enthaltende Monomere , die neben den monoethylenisch ungesättigtem, säuregruppenhaltigen Monomeren (α1) vorliegen können, sind Diallcylamrnoniumalkyl(meth)acrylate in quarternisierter Form, beispielsweise Trimethylammoniumethyl(meth)acrylat-Methosulfat oder Dimethylethylammoniumethyl(meth)acrylat-Ethosulfat sowie (Meth)acrylamido-alkyldialkylamine in quarternisierter Form, beispielsweise (Meth)acrylamidopropyltrimethylammoniumchlorid, Trimethylammoniumethyl-(meth)acrylat-Chlorid oder (Meth)acrylamidopropyltrimethylammoniumsulfat bevorzugt.

Als monoethylenisch ungesättigte, mit (α1) copolymerisierbare Monomere (α2) sind Acrylamide und Methacrylamide bevorzugt.

Mögliche (Meth)acrylamide sind neben Acrylamid und Methacrylamid alkylsubstituierte (Meth)acrylamide oder aminoalkylsubstituierte Derivate des (Meth)acrylamids, wie N-Methylol(meth)acrylamid, N,N-Dimethylamino(meth)acrylamid, Dimethyl(ineth)acrylamid oder Diethyl(meth)acrylamid Mögliche Vinylamide sind beispielsweise N-Vinylamide, N-Vinylformamide, N-Vinylacetamide, N-Vinyl-N-Methylacetamide, N-Vinyl-N-methylformamide, Vinylpyrrolidon. Unter diesen Monomeren besonders bevorzugt ist Acrylamid.

Zudem sind als monoethylenisch ungesättigte, mit (α1) copolymerisierbaren Monomere (α2) in Wasser dispergierbare Monomere bevorzugt. Als in Wasser dispergierbare Monomere sind Acrylsäurester und Methacrylsäurester, wie Methyl(meth)acrylat, Ethyl(meth)acrylat, Propyl(meth)acrylat oder Butyl(meth)acrylat, sowie Vinylacetat, Styrol und Isobutylen bevorzugt.

Erfindungsgemäß bevorzugte Vernetzer (α3) sind Verbindungen, die mindestens zwei ethylenisch ungesättigte Gruppen innerhalb eines Moleküls aufweisen (Vernetzerklasse I), Verbindungen, die mindestens zwei funktionelle Gruppen aufweisen, die mit funktionellen Gruppen der Monomeren (α1) oder (α2) in einer Kondensationsreaktion (=Kondensationsvernetzer), in einer Additionsreaktion oder in einer Ringöffnungsreaktion reagieren können (Vernetzerklasse II), Verbindungen, die mindestens eine ethylenisch ungesättigte Gruppe und mindestens eine funktionelle Gruppe, die mit funktionellen Gruppen der Monomeren (α1) oder (α2) in einer Kondensationsreaktion, in einer Additionsreaktion oder in einer Ringöffnungsreaktion reagieren kann (Vernetzerklasse III), aufweisen, oder polyvalente Metallkationen (Vernetzerklasse IV). Dabei wird durch die Verbindungen der Vernetzerklasse I eine Vernetzung der Polymere durch die radikalische Polymerisation der ethylenisch ungesättigten Gruppen des Vernetzermoleküls mit den monoethylenisch ungesättigten Monomeren (α1) oder (α2) erreicht, während bei den Verbindungen der Vernetzerklasse II und den polyvalenten Metallkationen der Vernetzerklasse IV eine Vernetzung der Polymere durch Kondensationsreaktion der funktionellen Gruppen (Vernetzerklasse II) bzw. durch elektrostatische Wechselwirkung des polyvalenten Metallkations (Vernetzerklasse IV) mit den funktionellen Gruppen der Monomere (α1) oder (α2) erreicht wird. Bei den Verbindungen der Vernetzerklasse III erfolgt dementsprechend eine Vernetzung des Polymers sowohl durch radikalische Polymerisation der ethylenisch ungesättigten Gruppe als auch durch Kondensationsreaktion zwischen der funktionellen Gruppe des Vernetzers und den funktionellen Gruppen der Monomeren (α1) oder (α2).

Bevorzugte Verbindungen der Vernetzerklasse I sind Poly(meth)acrylsäureester, die beispielsweise durch die Umsetzung eines Polyols, wie beispielsweise Ethylenglykol, Propylenglykol, Trimethylolpropan, 1,6-Hexandiol, Glycerin, Pentaerythrit, Polyethylenglykol oder Polypropylenglykol, eines Aminoalkohols, eines Polyalkylenpolyaminens, wie beispielsweise Diethylentriamin oder Triethylentetraamin, oder eines alkoxylierten Polyols mit Acrylsäure oder Methacrylsäure gewonnen werden. Als Verbindungen der Vernetzerklasse I sind des Weiteren Polyvinylverbindungen, Poly(meth)allyverbindungen, (Meth)acrylsäureester einer Monovinylverbindung oder (Meth)acrylsäureester einer Mono(meth)allylverbindung, vorzugsweise der Mono(meth)allylverbindungen eines Polyols oder eines Aminoalkohols, bevorzugt. In diesem Zusammenhang wird auf DE 195 43 366 und DE 195 43 368 verwiesen.

Bevorzugte Vernetzer der Vernetzerklasse II, III und IV sind diejenigen Vernetzer, die in WO 01/41819 offenbart sind und die die vorstehend beschriebenen Eigenschaften der Vernetzer der Vemetzerklassen II, III und IV erfüllen.

Als wasserlösliche Polymere (α4) können in den erfindungsgemäßen wasserabsorbierenden Polymer wasserlösliche Polymerisate, wie teil- oder vollverseifter Polyvinylalkohol, Polyvinylpyrrolidon, Stärke oder Stärkederivate, Polyglykole oder Polyacrylsäure enthalten, vorzugsweise einpolymerisiert sein. Das Molekulargewicht dieser Polymere ist unkritisch, solange sie wasserlöslich sind. Bevorzugte wasserlösliche Polymere sind Stärke oder Stärkederivate oder Polyvinylalkohol. Die wasserlöslichen Polymere, vorzugsweise synthetische wie Polyvinylalkohol, können auch als Pfropfgrundlage für die zu polymerisierenden Monomeren dienen.

Als Hilfsstoffe (α5) werden vorzugsweise Stellmittel, Geruchsbinder, oberflächenaktive Mittel oder Antioxidatien eingesetzt.

Als Zusatzstoffe eignen sich insbesondere Stoffe, die zur Stabilisierung des Silberions oder des Silbersalzes beitragen. Von besonderem Interesse sind hierbei Stoffe, die durch Silber bedingte Verfärbung des erfindungsgemäßen wasserabsorbierenden antimikrobiellen Polymers unterbinden oder zumindest aufhalten können.

Weiterhin können, insbesondere zum Verhindern des Auswaschens von Silberionen oder von sich bildenden elementaren Silber weitere Verbindungen in die erfindungsgemäßen wasserabsorbierenden, antimikrobiellen Polymerteilchen eingearbeitet werden. Hierunter sind Tenside bevorzugt, wie sie beispielsweise in WO 01/41819 beschrieben sind.

Gemäß einer bevorzugten Ausführungsform liegen die erfindungsgemäßen wasserabsorbierenden, antimikrobiellen Polymerteilchen als im wesentlichen kugelförmige oder pelletartige Teilchen vor, die eine Konzentration eines auf einem Silbersalz basierenden Silberions im Bereich von 1 bis 500 ppm, bevorzugt im Bereich von 5 bis 300 ppm und besonders bevorzugt im Bereich von 10 bis 70 ppm aufweisen.

Es ist erfindungsgemäß weiterhin bevorzugt, dass die wasserabsorbierenden, antimikrobiellen Polymerteilchen einen Innenbereich, einen den Innenbereich umgebenden Aussenbereich sowie einen den Aussenbereich umgebenden Oberflächenbereich aufweisen, wobei der Aussenbereich einen höheren Vernetzungsgrad als der Innenbreich aufweist, so dass sich vorzugsweise eine Kern-Schale-Struktur ausbildet. Die erhöhte Vernetzung im Oberflächenbereich der Polymerteilchen wird dabei vorzugsweise durch Nachvernetzung oberflächennaher, reaktiver Gruppen erreicht. Diese Nachvernetzung kann thermisch, photochemisch oder chemisch erfolgen. Genauere Angaben über die Nachvemetzungsbedingungen können den nachfolgenden Ausführungen über die erfindungsgemäßen Verfahren zur Herstellung wasserabsorbierender, antimikrobieller Polymerteilchen entnommen werden.

Weiterhin betrifft die Erfindung ein Verfahren zur Herstellung eines wasserabsorbierenden antimikrobiellen Polymerteilchens, wobei als Edukte
(β1) 50 bis 99,99 Gew.-%, vorzugsweise 70 bis 99,99 Gew.-% und besonders bevorzugt 85 bis 99,99 Gew.-% polymerisierten, ethylenisch ungesättigten, säuregruppenhaltigen Monomeren oder deren Salze oder deren Mischungen,
(β2) 0 bis 40 Gew.-%, bevorzugt 1 bis 30 Gew.-% und besonders bevorzugt 5 bis 20 Gew.-% polymerisierten, monoethylenisch ungesättigten, mit (α1) polymerisierbaren Monomeren,
(β3) 0,01 bis 5 Gew.-%, vorzugsweise 0,1 bis 3 Gew.-% und besonders bevorzugt 0,5 bis 2 Gew.-% eines oder mehrerer Vernetzer, wobei diese Vernetzer Verbindungen sind, die mindestens zwei ethylenisch ungesättigte Gruppen innerhalb eines Moleküls aufweisen (Vernetzerklasse I), Verbindungen, die mindestens zwei funktionelle Gruppen aufweisen, die mit funktionellen Gruppen der Monomeren (α1) oder (α2) in einer Kondensationsreaktion, in einer Additionsreaktion oder in einer Ringöffnungsreaktion reagieren können (Vernetzerklasse II), Verbindungen, die mindestens eine ethylenisch ungesättigte Gruppe und mindestens eine funktionelle Gruppe, die mit funktionellen Gruppen der Monomeren (α1) oder (α2) in einer Kondensationsreaktion, in einer Additionsreaktion oder in einer Ringöffnungsreaktion reagieren kann (Vernetzerklasse III), aufweisen, oder polyvalente Metallkationen (Vernetzerklasse IV),
(β4) 0 bis 30 Gew.-%, bevorzugt 1 bis 20 Gew.-% und besonders bevorzugt 5 bis 10 Gew.-% eines wasserlöslichen Polymeren sowie
(β5) 0 bis 20 Gew.-%, bevorzugt 0,01 bis 7 Gew.-% und besonders bevorzugt 0,05 bis 5 Gew.-% eines oder mehrerer Hilfsstoffe,
wobei die Summe der Gewichtsmengen (β1) bis (β5) 100 Gew.-% beträgt, miteinander unter Bildung eines wasserabsorbierenden Polymers polymerisiert werden;
wobei 1 bis 500 ppm, bevorzugt 5 bis 300 ppm eines Silberions in Form eines wasserlöslichen Silbersalzes, welches ein Löslichkeitsprodukt, vorzugsweise ein Löslichkeitsprodukt in Wasser, besonders bevorzugt ein Löslichkeitsprodukt in Wasser bei einer Temperatur von 25°C, von mindestens 1 × 10⁻⁸ (Mol/l)^{m+n}, vorzugsweise von mindestens 1 × 10⁻⁷ (Mol/l)^{m+n}, besonders bevorzugt von mindestens 1 × 10⁻⁶ (Mol/l)^{m+n}, darüber hinaus bevorzugt von mindestens 1 × 10⁻⁵ (Mol/l)^{m+n}, darüber hinaus noch mehr bevorzugt von mindestens 1 × 10⁻⁴ (Mol/1)^{m+n} und am meisten bevorzugt von mindestens 1 × 10⁻³ (Mol/l)^{m+n} besitzt, in einem Lösemittel gelöst, bezogen auf die Edukte, vor Abschluss der Bildung des absorbierenden Polymers den Edukten zugesetzt wird, und wobei
X in der Formel Silbersalz AgₘXₙ für ein Anion aus der Gruppe von Nitrat, Carbonat, Sulfat, Hydrogensulfat, Alaun, Phosphat, Acrylat, Lactat, Acetat, Sulfonat, Benzoat, Triflourmethansulfonat oder Acrylat,
und wobei das wasserabsorbierende Polymer zerkleinert, getrocknet und gegebenenfalls gemahlen wird und in einem Nachvernetzungsschritt mit einem Nachvernetzer, wobei dieser Nachvernetzer aus der Gruppe von Diethylenglykol, Triethylenglykol, Polyethylenglykol, Glyzerin, Polyglyzerin, Propylenglykol, Diethanolamin, Triethanolamin, Polyoxypropylen, Oxyethylen-Oxypropylen-Blockcopolymere, Sorbitanfettsäureester, Polyoxyethylen-sorbitanfettsäureester, Trimethylolpropan, Pentaerytrit, Polyvinylalkohol, Sorbitol, 1,3-Dioxolan-2-on (Ethylencarbonat), 4-Methyl-1,3-dioxolan-2-on (Propylencarbonat), 4,5-Dimethyl-1,3-dioxolan-2-on, 4,4-Dimethyl-1,3-dioxolan-2-on, 4-Ethyl-1,3-dioxolan-2-on, 4-Hydroxymethyl-1,3-dioxolan-2-on, 1,3-Dioxan-2-on, 4-Methyl-1,3-dioxan-2-on, 4,6-Dimethyl-1,3-dioxan-2-on, 1,3-Dioxolan-2-on, Poly-1,3-dioxolan-2-on ausgewählt ist, und in einer Menge in einem Bereich von 0,01 bis 30 Gew.-%, bezogen auf das noch unbehandelte, vorzugsweise des noch nicht mit dem Nachvernetzer versetzten Polymerteilchens, nachvernetzt wird.

Weiterhin betrifft die Erfindung ein Verfahren zur Herstellung eines wasserabsorbierenden antimikrobiellen Polymerteilchens, wobei als Edukte
(β1) 50 bis 99,99 Gew.-%, vorzugsweise 70 bis 99,99 Gew.-% und besonders bevorzugt 85 bis 99,99 Gew.-% polymerisierten, ethylenisch ungesättigten, säuregruppenhaltigen Monomeren oder deren Salze oder deren Mischungen,
(β2) 0 bis 40 Gew.-%, bevorzugt 1 bis 30 Gew.-% und besonders bevorzugt 5 bis 20 Gew.-% polymerisierten, monoethylenisch ungesättigten, mit (α1) polymerisierbaren Monomeren,
(β3) 0,01 bis 5 Gew.-%, vorzugsweise 0,1 bis 3 Gew.-% und besonders bevorzugt 0,5 bis 2 Gew.-% eines oder mehrerer Vernetzer,
(β4) 0 bis 30 Gew.-%, bevorzugt 1 bis 20 Gew.-% und besonders bevorzugt 5 bis 10 Gew.-% eines wasserlöslichen Polymeren sowie
(β5) 0 bis 20 Gew.-%, bevorzugt 0,01 bis 7 Gew.-% und besonders bevorzugt 0,05 bis 5 Gew.-% eines oder mehrerer Hilfsstoffe,
wobei die Summe der Gewichtsmengen (β1) bis (β5) 100 Gew.-% beträgt, miteinander unter Bildung eines wasserabsorbierenden Polymers polymerisiert werden; wobei 1 bis 500 ppm, bevorzugt 5 bis 300 ppm eines Silberions in Form eines wasserlöslichen Silbersalzes, welches ein Löslichkeitsprodukt, vorzugsweise ein Löslichkeitsprodukt in Wasser, besonders bevorzugt ein Löslichkeitsprodukt in Wasser bei einer Temperatur von 25°C, von mindestens 1 × 10⁻⁸ (Mol/l)^{m+n}, vorzugsweise von mindestens 1 × 10⁻⁷ (Mol/l)^{m+n}, besonders bevorzugt von mindestens 1 × 10⁻⁶ (Mol/l)^{m+n}, darüber hinaus bevorzugt von mindestens 1 × 10⁻⁵ (Mol/l)^{m+n}, darüber hinaus noch mehr bevorzugt von mindestens 1 × 10⁻⁴ (Mol/l)^{m+n} und am meisten bevorzugt von mindestens 1 × 10⁻³ (Mol/l)^{m+n} besitzt, in einem Lösemittel gelöst, bezogen auf die Edukte, vor Abschluss der Bildung des absorbierenden Polymers den Edukten zugesetzt wird,
und wobei die ethylenisch ungesättigten, säuregruppenhaltigen Monomere (β1) dergestalt neutralisiert werden, das der pH-Wert der wasserabsorbierenden antimikrobiellen Polymerteilchen im Bereich von 4,5 bis 7 liegt.

Gemäß einer bevorzugten Ausführungsform der erfindungsgemäßen Verfahren wird das Silbersalz in dem gleichen Lösemittel gelöst, in dem die Monomere gelöst werden. Dabei ist es nach einer Variante der erfindungsgemäßen Verfahren bevorzugt, dass das Silbersalz dem Lösemittel, in dem die Monomere anschließend aufgenommen werden, zuvor zugesetzt wird. Im allgemeinen wird die Menge des Silbersalzes, das dem Lösemittel zugesetzt wird, so gewählt, dass die Konzentration des auf dem Silbersalz basierenden Silberions in dem fertigen wasserabsorbierenden antimikrobiellen Polymerteilchen 1 bis 500 ppm beträgt. Vorzugsweise liegt die Konzentration des Silbersalzes im Bereich von 0,003 bis 0,5 Gramm pro Liter Lösemittel, vorzugsweise im Bereich von 0,003 bis 0,1 Gramm pro Liter Lösemittel, und besonders bevorzugt im Bereich von 0,005 bis 0,05 Gramm pro Liter Lösemittel, das anschließend zur Lösung der Monomere eingesetzt wird. In einer anderen Variante des erfindungsgemäßen Verfahrens wird das Silbersalz in einem Lösemittel gelöst und die so entstehende Silbersalzlösung der Monomerlösung zugesetzt. Auch in diesem Fall werden die Konzentrationsverhältnisse in Abhängigkeit von der Lösemittelmenge, die zur Aufnahme der Monomere genutzt wird, so gewählt, dass bei den fertigen wasserabsorbierenden antimikrobiellen Polymerteilchen die Konzentration des auf einem Silbersalz basierenden Silberions im Bereich von 1 bis 500 ppm liegt.

Als Lösemittel können alle dem Fachmann geeigneten Lösemittel eingesetzt werden, in denen die in dem erfindungsgemäßen Verfahren eingesetzten Silbersalze leicht löslich sind. Als Lösemittel kommen insbesondere Wasser und niedere Alkohole, insbesondere Methanol, Ethanol, Propanol, Isopropanol und Butanol in Betracht, wobei es erfindungsgemäß bevorzugt ist, dass das Lösemittel zumindest 50 Gew.-%, bevorzugt mindestens 70 Gew.-% und besonders bevorzugt mindestens 90 Gew.-%, bezogen auf das Lösemittel, auf Wasser, basiert.

Die Polymerisation der erfindungsgemäßen Verfahren gilt dann als abgeschlossen, wenn der Restmonomergehalt unter 1 %, bezogen auf die eingesetzten Monomere, liegt. Im erfindungsgemäßen Verfahren kann das in einem Lösemittel gelöste Silbersalz bevorzugt bis zu einem Restmonomergehalt von 10 Gew.-%, besonders bevorzugt bis zu einem Restmonomergehalt von 50 Gew.-% und darüber hinaus bevorzugt bis zu einem Restmonomergehalt von 80 Gew.-% sowie noch bevorzugter vor dem Beginn der Polymerisationsreaktion, d.h. bei einem Restmonomergehalt von 100 Gew.-%, jeweils bezogen auf die eingesetzten Monomere, den Edukten zugesetzt werden.

Durch die erfindungsgemäße Verfahrensführung wird eine möglichst homogene Verteilung der auf einem Silbersalz basierenden Silberionen erreicht.

Bezüglich der Edukte (β1) bis (β5) wird auf die Ausführungen zu (α1) bis (α5) verwiesen.

Aus den vorgenannten Monomeren und Vernetzern lassen sich die erfindungsgemäßen wasserabsorbierende Polymere durch verschiedene Polymerisationsweisen herstellen. Beispielsweise sind in diesem Zusammenhang Massepolymerisation, die vorzugsweise in Knetreaktoren wie Extrudern oder durch Bandpolymerisation erfolgt, Lösungspolymerisation, Spraypolymerisation, inverse Emulsionspolymerisation und inverse Suspensionspolymerisation zu nennen. Bevorzugt wird die Lösungspolymerisation in Wasser als Lösemittel durchgeführt. Die Lösungspolymerisation kann kontinuierlich oder diskontinuierlich erfolgen. Aus dem Stand der Technik ist ein breites Spektrum von Variationsmöglichkeiten hinsichtlich Reaktionsverhältnisse wie Temperaturen, Art und Menge der Initiatoren als auch der Reaktionslösung zu entnehmen. Typische Verfahren sind in den folgenden Patentschriften beschrieben: US 4,286,082, DE 27 06 135, US 4,076,663, DE 35 03 458, DE 40 20 780, DE 42 44 548, DE 43 23 001, DE 43 33 056, DE 44 18 818.

Besonders bevorzugt lassen sich die erfindungsgemäßen Verfahren bei der Bandpolymerisation des wasserabsorbierenden antimikrobiellen Polymers einsetzen. In der Regel weist das in diesen Verfahren eingesetzte Muldenband nur wenige oder keine Rührmittel wie Rührer oder Knetharken auf. Somit kann durch das erfindungsgemäße Verfahren eine gute Verteilung des auf einem Silbersalz basierenden Silberions ohne gesonderte Rührmittel erreicht werden.

Eine andere Möglichkeit zur Herstellung der erfindungsgemäßen wasserabsorbierenden Polymere besteht darin, zunächst unvernetzte, insbesondere lineare Polymere, vorzugsweise auf radikalischem Wege aus den vorgenannten monoethylenisch ungesättigten Monomeren (α1) bzw. (α2) (bzw. (β1) und (β2)) herzustellen und diese dann mit vernetzend wirkenden Reagenzien (α3) (bzw. (β3)), vorzugsweise denen der Klassen II und IV, umzusetzen. Diese Variante wird vorzugsweise dann eingesetzt, wenn die wasserabsorbierenden Polymere zunächst in formgebenden Verfahren, beispielsweise zu Fasern, Folien oder anderen Flächengebilden, wie Geweben, Gewirken, Gespinsten oder Vliesen verarbeitet und in dieser Form vernetzt werden sollen.

Weiterhin ist es bei den erfindungsgemäßen Verfahren bevorzugt, das wasserabsorbierende Polymer zu zerkleinern, zu trocknen und gegebenenfalls zu mahlen und das derartig weiterverarbeitete wasserabsorbierende Polymer in einem sogenannten "Nachvernetzungsschritt" mit weiteren Vernetzern, sogenannten "Nachvernetzern", zu versetzen und gegebenenfalls nochmals thermisch zu behandeln.

Als Nachvernetzer bevorzugt sind die im Zusammenhang mit den Vernetzern genannten Verbindungen der Vernetzerklasse II und IV.

Unter diesen Verbindungen sind als Nachvernetzer besonders bevorzugt Diethylenglykol, Triethylenglykol, Polyethylenglykol, Glyzerin, Polyglyzerin, Propylenglykol, Diethanolamin, Triethanolamin, Polyoxypropylen, Oxyethylen-Oxypropylen-Blockcopolymere, Sorbitanfettsäureester, Polyoxyethylensorbitanfettsäureester, Trimethylolpropan, Pentaerytrit, Polyvinylalkohol, Sorbitol, 1,3-Dioxolan-2-on (Ethylencarbonat), 4-Methyl-1,3-dioxolan-2-on (Propylencarbonat), 4,5-Dimethyl-1,3-dioxolan-2-on, 4,4 Dimethyl-1,3-dioxolan-2-on, 4-Ethyl-1,3-dioxolan-2-on, 4-Hydroxymethyl-1,3-dioxolarl-2-on, 1,3-Dioxan-2-on, 4-Methyl-1,3-dioxan-2-on, 4,6-Dimethyl-1,3-dioxan-2-on, 1,3-Dioxolan-2-on, Poly-1,3-dioxolan-2-on.

Besonders bevorzugt wird Ethylencarbonat als Nachvernetzer eingesetzt.

Diese Verbindungen werden vorzugsweise in einer Menge im Bereich von 0,01 bis 30, bevorzugt 0,1 bis 20 und besonders bevorzugt 0,5 bis 10 Gew.-%, bezogen auf das noch unbehandelte, vorzugsweise auf das noch nicht mit dem Nachvernetzer versetzte Polymer, eingesetzt. Organische Lösemittel können der Mischung in einer Menge von 0 bis 60, bevorzugt 0,1 bis 40 und besonders bevorzugt 0,2 bis 50 Gew.-%, bezogen auf das noch unbehandelte Polymer, zugesetzt werden. Als organische Lösemittel sind bevorzugt niedere Alkohole wie Methanol, Ethanol, n-Propanol, Isopropanol, n-Butanol, Isobutanol, sec-Butanol und t-Butanol, Ketone wie Aceton, Methylethylketon und Methylisobutylketon, Ether wie Dioxan, Tetrahydrofuran und Diethylether, Amide wie N,N-Dimethylformamid und N,N-Diethylformamid sowie Sulfoxide wie Dimethylsulfoxid.

Weiterhin ist es für das erfindungsgemäße wasserabsorbierende antimikrobielle Polymerteilchen und die Verfahren in einer Ausführungsform bevorzugt, dass 30 bis 80 Mol-%, vorzugsweise 40 bis 70 Mol % und besonders bevorzugt 50 bis 65 Mol-% der Säuregruppen aus den ethylenisch ungesättigten, säuregruppehaltigen Monomer des wasserabsorbierenden Polymers mit einem Alkali- oder Erdalkalisalz, vorzugsweise einem Alkalisalz und besonders bevorzugt Natriumhydroxid, neutralisiert wird. Die Neutralisation erfolgt erfindungsgemäß bevorzugt dergestalt, dass der pH-Wert des erfindungsgemäßen wasserabsorbierenden antimikrobiellen Polymerteilchen im Bereich von 4,5 bis 7, bevorzugt von 5,2 bis 6,5 und besonders bevorzugt von 5,3 bis 6,2 liegt. Durch eine derartige Neutralisation wird zum einen die Hautverträglichkeit des erfindungsgemäßen wasserabsorbierenden antimikrobiellen Polymerteilchens erhöht und zudem die Geruchsbindungsfähigkeit des erfindungsgemäßen wasserabsorbierenden antimikrobiellen Polymerteilchens verbessert.

Außerdem ist es für die erfindungsgemäßen wasserabsorbierenden, antimikrobiellen Polymerteilchen und die erfindungsgemäßen Verfahren bevorzugt, dass das Silbersalz Ag^{m}Xⁿ ein Löslichkeitsprodukt, vorzugsweise in Wasser, 1 × 10⁻⁸ (Mol/l)^{m+n}, vorzugsweise von mindestens 1 × 10⁻⁷ (Mol/l)^{m+n}, besonders bevorzugt von mindestens 1 × 10⁻⁶ (Mol/l)^{m+n}, darüber hinaus bevorzugt von mindestens 1 × 10⁻⁵ (Mol/l)^{m+n}, darüber hinaus noch mehr bevorzugt von mindestens 1 × 10⁻⁴ (Mol/l)^{m+n} und am meisten bevorzugt von mindestens 1 × 10⁻³ (Mol/l)^{m+n} besitzt. In einer besonderen Ausführungsform der erfindungsgemäßen Verfahren bzw. der erfindungsgemäßen wasserabsorbierenden, antimikrobiellen Polymerteilchen wird ein Silbersalz eingesetzt bzw. ist ein Silbersalz enthalten, von dem bei einer Temperatur von 25°C mindestens 1g, vorzugsweise mindestens 5 g, besonders bevorzugt mindestens 10 g, darüber hinaus bevorzugt mindestens 50 g, darüber hinaus noch mehr bevorzugt mindestens 100 g und am meisten bevorzugt mindestens 200 g in 100 g Wasser vollständig gelöst werden können.

Ferner ist es bevorzugt, dass bei den erfindungsgemäßen wasserabsorbierenden, antimikrobiellen Polymerteilchen und in den erfindungsgemäßen Verfahren das Silbersalz Silbernitrat ist. Neben Silbernitrat können andere anorganische oder organische Silbersalze verwendet werden. In diesem Zusammenhang sind als anorganische Silbersalze Silbercarbonat, Silbersulfat, Silberhydrogensulfat, Silberalaun oder Silberphosphat beispielsweise zu nennen. Als organische Silbersalze kommen Silberacrylat, Silbercitrat, Silberlactat, Silberacetat, Silbertoluolsulfonat, Silberbenzoat, Silbertriflourmethansulfonat oder im Fall des Silberacrylat dessen Polymere in Betracht. Jedes der vorstehend genannten Silbersalze kann für sich in den erfindungsgemäßen antimikrobiellen, wasserabsorbierenden Polymerteilchen enthalten sein bzw. in den erfindungsgemäßem Verfahren eingesetzt werden und damit eine bevorzugte Ausführungsform der erfindungsgemäßen wasserabsorbierenden, antimikrobiellen Polymerteilchen bzw. der erfindungsgemäßen Verfahren darstellen. Silbernitrat-beinhaltende erfindungsgemäße antimikrobielle, wasserabsorbierende Polymerteilchen sind von diesen am meisten bevorzugt.

Zudem betrifft die Erfindung wasserabsorbierende, antimikrobielle Polymerteilchen, die durch die vorstehenden Verfahren erhältlich sind. Vorzugsweise weisen die durch die vorstehenden Verfahren erhältlichen wasserabsorbierenden, antimikrobiellen Polymerteilchen die gleichen Eigenschaften auf wie die eingangs beschriebenen, erfindungsgemäßen wasserabsorbierenden, antimikrobiellen Polymerteilchen.

Vorzugsweise weisen die erfindungsgemäßen wasserabsorbierenden, antimikrobiellen Polymerteilchen mindestens eine, vorzugsweise jede, der nachfolgenden Eigenschaften auf:
(A) maximale Aufnahme von 0,9 Gew.-%er NaCl-Lösung liegt nach ERT 440.1-99 in einem Bereich von mindestens 10 bis 1000 g/g, bevorzugt von 15 bis 500 und besonders bevorzugt von 20 bis 300 g/g;
(B) der mit 0,9 Gew.-%er wässriger NaCl-Lösung extrahierbare Anteil beträgt nach ERT 470.1-99 weniger als 30 Gew.-%, bevorzugt weniger als 20 Gew.-% und besonders bevorzugt weniger als 10 Gew.-%, jeweils bezogen auf das Polymer;
(C) die Schüttdichte liegt nach ERT 460.1-99 im Bereich von 300 bis 1000 g/l, bevorzugt von 310 bis 800 g/l und besonders bevorzugt von 320 bis 700 g/l;
(D) der pH-Wert von 1 g des Polymeren in 1 1 Wasser liegt gemäß ERT 400.1-99 im Bereich von 4 bis 10, bevorzugt von 4 bis 7, besonders bevorzugt von 5,2 bis 6,5 und darüber hinaus bevorzugt von 5,3 bis 6,2;
(E) die Centrifugation Retention Capacity (CRC) gemäß ERT 441.1-99 liegt im Bereich von 10 bis 100 g/g, bevorzugt von 15 bis 80 und besonders bevorzugt von 20 bis 60 g/g.

Die sich aus den vorstehenden Eigenschaften ergebenden Eigenschaftskombinationen von zwei oder mehr dieser Eigenschaften stellen jeweils bevorzugte Ausführungsformen der erfindungsgemäßen Polymerteilchen dar. Weiterhin als erfindungsgemäße Ausführungsformen besonders bevorzugt sind wasserabsorbierende antimikrobielle Polymerteilchen, die die nachfolgend als Buchstaben oder Buchstabenkombinationen dargestellten Eigenschaften oder Eigenschaftskombinationen zeigen: A, B, C, D, E, AB, ABC, ABCD, ABCDE, BC, BCD, BCDE, CD, CDE, DE.

Gemäß einer bevorzugten Ausführungsform weisen die erfindungsgemäßen wasserabsorbierenden, antimikrobiellen Polymerteilchen eine antimikrobielle Wirkung auf, bei der die Menge an NH₃ / h nach mindestens 6, vorzugsweise mindestens 12 und besonders bevorzugt mindestens 24 Tagen 200 ppm nicht übersteigt, die nach der nachfolgend unter Testmethoden und Beispielen angegebnen Verfahren erhalten wird.

Die Erfindung betrifft auch einen Verbund umfassend erfindungsgemäße wasserabsorbierende, antimikrobielle Polymerteilchen sowie ein Substrat. Es ist bevorzugt, dass die erfindungsgemäßen wasserabsorbierenden, antimikrobiellen Polymerteilchen und das Substrat miteinander fest verbunden sind. Als Substrate sind Folien aus Polymeren, wie beispielsweise aus Polyethylen, Polypropylen oder Polyamid, Metalle, Vliese, Fluff, Tissues, Gewebe, natürliche oder synthetische Fasern, oder andere Schäume bevorzugt.

Wenn der Verbund ein absorbierendes Core ist, ist das wasserabsorbierende, antimikrobielle Polymerteilchen in ein Substrat eingearbeitet. Bei diesem Substrat handelt es sich vorzugsweise um Fasermaterialien. Fasernmaterialien, die in der vorliegenden Erfindung verwendet werden können, umfassen natürlich vorkommende Fasern (modifiziert oder nicht modifiziert) als auch synthetische Fasern. Beispiele geeigneter nicht modifizierter und modifizierter natürlich vorkommender Fasern umfassen Baumwolle, Espartogras, Zuckerrohr, Grannenhaar, Flachs, Seide, Wolle, Zellstoff, chemisch modifizierter Zellstoff, Jute, Reyon, Ethylzellulose und Zelluloseacetat. Geeignete synthetische Fasern können aus Polyvinylchlorid, Polyvinylfluorid, Polytetrafluorethylen, Polyvinylidenchlorid, Polyacrylat wie Orion®, Polyvinylacetat, Polyethylvinylacetat, nicht löslichem oder löslichem Polyvinylalkohol, Polyolefine, wie Polyethylen (beispielsweise PULPEX®) und Polypropylenen, Polyamiden, wie Nylon, Polyestern wie DACRON® oder Kodel®, Polyurethanen, Polystyrolen und dergleichen hergestellt werden. Die verwendeten Fasern können nur natürlich vorkommende Fasern, nur synthetische Fasern oder irgend eine kompatible Kombination aus natürlich vorkommenden und synthetischen Fasern umfassen. Die wasserlöslichen antimikrobielle Polymerteilchen werden durch die Struktur des Substrats oder durch ein Haftmittel oder durch beides in dem Substrat gehalten.

Die in der vorliegenden Erfindung verwendeten Fasern können hydrophil oder hydrophob sein, oder sie können aus einer Kombination aus hydrophilen und hydrophoben Fasern bestehen. Der Ausdruck "hydrophil", wie er hier verwendet wird, beschreibt Fasern oder Oberflächen von Fasern, die durch wässrige Flüssigkeiten (beispielsweise wässrige Körperflüssigkeiten), die auf diesen Fasern abgesetzt sind, benetzbar sind. Hydrophilie und Benetzbarkeit werden typischerweise in Ausdrücken des Kontaktwinkels und der Oberflächenspannung der beteiligten Flüssigkeiten und Feststoffe definiert. Dies wird im Detail in einer Veröffentlichung der American Chemical Society mit dem Titel "Contact Angle, Wettability and Adhesion", herausgegeben von Robert F. Gould (Copyright 1964) diskutiert. Eine Faser oder die Oberfläche einer Faser wird durch eine Flüssigkeit benetzt

(das heißt sie ist hydrophil), wenn entweder der Kontaktwinkel zwischen der Flüssigkeit und der Faser oder dessen Oberfläche weniger als 90° beträgt, oder wenn die Flüssigkeit dazu neigt, sich spontan über der Oberfläche zu verteilen, wobei beide Bedingungen normalerweise gleichzeitig vorliegen. Umgekehrt wird eine Faser oder die Oberfläche einer Faser als hydrophob betrachtet, wenn der Kontaktwinkel größer als 90° ist und die Flüssigkeit sich nicht spontan auf der Oberfläche der Faser ausbreitet.

In einer erfindungsgemäß besonders bevorzugten Ausführungsform ist der Verbund eine Windel. Hierbei stellen die Bestandteile der Windel, die von dem erfindungsgemäßen wasserabsorbierenden antimikrobiellen Polymerteilchen verschieden sind, das Substrat des Verbundes dar. In einer bevorzugten Ausführungsform enthält die Windel ein zuvor beschriebenes Core. In diesem Fall stellen die von dem Core unterschiedlichen Bestandteile der Windel das Substrat des Verbundes dar. Im allgemeinen umfasst ein als Windel eingesetzter Verbund eine wasserundurchlässige Unterschicht, eine wasserdurchlässige, vorzugsweise hydrophobe, Oberschicht und eine das erfindungsgemäße absorbierende, schaumförmige Polymergebilde beinhaltende Schicht, die zwischen der Unterschicht und der Oberschicht angeordnet ist. Diese das erfindungsgemäße absorbierenden, schaumförmige Polymergebilde beinhaltende Schicht ist vorzugsweise ein zuvor beschriebenes Core. Die Unterschicht kann alle dem Fachmann bekannten Materialien aufweisen, wobei Polyethylen oder Polypropylen bevorzugt sind. Die Oberschicht kann gleichfalls alle dem Fachmann bekannten und geeigneten Materialien enthalten, wobei Polyester, Polyolefine, Viskose und dergleichen bevorzugt sind, die eine so poröse Schicht ergeben, die einen ausreichenden Flüssigkeitsdurchlass der Oberschicht sicherstellen. In diesem Zusammenhang wird auf die Offenbarung in US 5,061,295, US Re. 26,151, US 3,592,194, US 3,489,148 sowie US 3,860,003 verwiesen. Ferner betrifft die Erfindung die Verwendung eines erfindungsgemäßen wasserabsorbierenden, antimikrobiellen Polymerteilchens in Fasern, Folien, Schäumen, Formkörpern oder Verbunden. Hierbei ist es bevorzugt, dass die erfindungsgemäßen wasserabsorbierenden, antimikrobiellen Polymerteilchen in Fasern, Folien, Schäumen, Formkörpern eingearbeitet werden.

Zudem betrifft die Erfindung die Verwendung eines erfindungsgemäßen Polymerteilchens; oder von erfindungsgemäßen Fasern, Folien, Schäumen oder Formkörpern; oder eines erfindungsgemäßen Verbunds; oder mindestens zwei davon, zur Herstellung eines Hygieneartikels, insbesondere einer Wundauflage, Windel, Damenbinde oder eines Inkontinenzartikels, zur Vorbeugung oder Behandlung von durch Körperausscheidungen hervorgerufenen Hautirritationen, vorzugsweise Windeldermatitis oder Agyose; oder zur Behandlung von Wunden.

Die nachfolgenden Beispiele sollen die Erfindung näher erläutern.

### TESTMETHODEN:

### Bestimmung der NH₃-Bildung

Ein Testbazillus wie der Bacillus pasteurii wird über Nacht bei 30°C in 100 ml Flüssigkultur im Schüttelwasserbad angezogen, 10 min bei Raumtemperatur zentrifugiert und in künstlichem Urin (Jayco Urintestlösung: Na₂SO₄ pa 2,00 g, KCl pa 2,00 g, NH₄H₂PO₄ pa 0,85g (NH₄)₂HPO₄ pa 0,15g MgCl2*6H₂O pa 0,50 g, CaCl₂*2H₂O 0,25 g, Aqua dest. 994,25 g) resuspendiert. Jeweils 33 ml des mit Bakterien versetzten künstlichen Urins werden in Erlenmeyerkolben überführt und mit 0,5 g Superabsorber versetzt. Die Gefäße werden mit einem Gummistopfen, durch dessen Bohrung ein Dräger-Diffusionsröhrchen geführt wird, verschlossen und bei 30°C in einen Brutschrank inkubiert.

Der freigesetzte Ammoniak wird in ppm x h gemessen. Als Kontrolle wird ein Ansatz ohne SAP mitgeführt. Alle Ansätze werden als Doppelbestimmung durchgeführt. Die dargestellten Ergebnisse ergeben sich aus den Mittelwerten.

### BEISPIELE:

### Beispiel 1

### (NG: 70; 0,03 g Silbernitrat)

In 955,985 g einer wässrigen Lösung von Natriumacrylat mit einem Neutralisationsgrad von 70 Mol-% (Monomer-Konzentration: 37,7 %) werden 0,45 g Polyethylenglycol-300-diacrylat und 1,05 g Polyethylenglycol-750-monoallyletheracrylat als Vernetzer gelöst. 0,03 g Silbernitrat werden in 10 g Wasser gelöst und in die Monomerenlösung gegeben. Anschließend wird die Monomerenlösung in einem Kunststoff-Polymerisationsgefäß für 30 Minuten mit Stickstoff durchspült, um den gelösten Sauerstoff zu entfernen. Bei einer Temperatur von 4 °C wird die Polymerisation durch die aufeinanderfolgende Zugabe von 0,3 g Natrium-peroxidisulfat in 10 g dest. Wasser, 0,1 g 2,2'-Azobis-2-amidinopropandihydrochlorid in 10 g dest. Wasser, 0,07 g 35 %ige Wasserstoffperoxidlösung in 10 g dest. Wasser und 0,015 g Ascorbinsäure in 2 g dest. Wasser gestartet. Nachdem die Endtemperatur (ca. 100 °C) erreicht ist, wird das Gel mit einem Fleischwolf zerkleinert und 2 h bei 150 °C in einem Umluftofen getrocknet. Das getrocknete Produkt wird grob zerstoßen, gemahlen und die Partikel der Größe 150 - 850 µm zur weiteren Umsetzung ausgesiebt (Pulver A).

50 g Pulver A werden unter kräftigem Rühren mit einer Lösung 0,5 g Ethylencarbonat und 1,5 g Wasser vermischt und danach für 60 min. in einem Ofen, der auf 170 °C temperiert ist, erhitzt.

### Beispiel 2

### (NG: 70; 0,012 g Silbernitrat)

In 955,997 g einer wässrigen Lösung von Natriumacrylat mit einem Neutralisationsgrad von 70 Mol-% (Monomer-Konzentration: 37,7 %) werden 0,45 g Polyethylenglycol-300-diacrylat und 1,05 g Polyethylenglycol-750-monoallyletheracrylat als Vernetzer gelöst. 0,012 g Silbernitrat werden in 10 g Wasser gelöst und in die Monomerenlösung gegeben. Anschließend wird die Monomerenlösung in einem Kunststoff-Polymerisationsgefäß für 30 Minuten mit Stickstoff durchspült, um den gelösten Sauerstoff zu entfernen. Bei einer Temperatur von 4 °C wird die Polymerisation durch die aufeinanderfolgende Zugabe von 0,3 g Natrium-peroxidisulfat in 10 g dest. Wasser, 0,1 g 2,2'-Azobis-2-amidinopropandihydrochlorid in 10 g dest. Wasser, 0,07 g 35 %ige Wasserstoffperoxidlösung in 10 g dest. Wasser und 0,015 g Ascorbinsäure in 2 g dest. Wasser gestartet. Nachdem die Endtemperatur (ca. 100 °C) erreicht ist, wird das Gel mit einem Fleischwolf zerkleinert und 2 h bei 150 °C in einem Umluftofen getrocknet. Das getrocknete Produkt wird grob zerstoßen, gemahlen und die Partikel der Größe 150-850 µm zur weiteren Umsetzung ausgesiebt (Pulver B).

50 g Pulver B wird unter kräftigem Rühren mit einer Lösung 0,5 g Ethylencarbonat und 1,5 g Wasser 1,5 g Wasser vermischt und danach für 60 min. in einem Ofen, der auf 170 °C temperiert ist, erhitzt.

### Vergleichsbeispiel 1

### (NG: 70; 0,014 g frisch gefälltes Silberchlorid)

In 955,985 g einer wässrigen Lösung von Natriumacrylat mit einem Neutralisationsgrad von 70 Mol-% (Monomer-Konzentration: 37,7 %) werden 0,45 g Polyethylenglycol-300-diacrylat und 1,05 g Polyethylenglycol-750-monoallyletheracrylat als Vernetzer gelöst. 0,014 g frisch gefälltes Silberchlorid werden in 10 g Wasser suspendiert und in die Monomerenlösung gegeben. Anschließend wird die Monomerenlösung in einem Kunststoff-Polymerisationsgefäß für 30 Minuten mit Stickstoff durchspült, um den gelösten Sauerstoff zu entfernen. Bei einer Temperatur von 4 °C wird die Polymerisation durch die aufeinanderfolgende Zugabe von 0,3 g Natrium-peroxidisulfat in 10 g dest. Wasser, 0,1 g 2,2'-Azobis-2-amidinopropandihydrochlorid in 10 g dest. Wasser, 0,07 g 35 %ige Wasserstoffperoxidlösung in 10 g dest. Wasser und 0,015 g Ascorbinsäure in 2 g dest. Wasser gestartet. Nachdem die Endtemperatur (ca. 100 °C) erreicht ist, wird das Gel mit einem Fleischwolf zerkleinert und 2 h bei 150 °C in einem Umluftofen getrocknet. Das getrocknete Produkt wurde grob zerstoßen, gemahlen und die Partikel der Größe 150 - 850 µm zur weiteren Umsetzung ausgesiebt (Pulver C).

50 g Pulver C wird unter kräftigem Rühren mit einer Lösung 0,5 g Ethylencarbonat und 1,5 g Wasser vermischt und danach für 60 min. in einem Ofen, der auf 170 °C temperiert ist, erhitzt.

### Vergleichsbeispiel 2

### (NG: 70; kein Silber)

In 965,988 g einer wässrigen Lösung von Natriumacrylat mit einem Neutralisationsgrad von 70 Mol-% (Monomer-Konzentration: 37,7 %) werden 0,45 g Polyethylenglycol-300-diacrylat und 1,05 g Polyethylenglycol-750-monoallyletheracrylat als Vernetzer gelöst. Anschließend wird die Monomerenlösung in einem Kunststoff-Polymerisationsgefäß für 30 Minuten mit Stickstoff durchspült, um den gelösten Sauerstoff zu entfernen. Bei einer Temperatur von 4 °C wird die Polymerisation durch die aufeinanderfolgende Zugabe von 0,3 g Natrium-peroxidisulfat in 10 g dest. Wasser, 0,1 g 2,2'-Azobis-2-amidinopropandihydrochlorid in 10 g dest. Wasser, 0,07 g 35 %ige Wasserstoffperoxidlösung in 10 g dest. Wasser und 0,015 g Ascorbinsäure in 2 g dest. Wasser gestartet. Nachdem die Endtemperatur (ca. 100 °C) erreicht ist, wird das Gel mit einem Fleischwolf zerkleinert und 2 h bei 150 °C in einem Umluftofen getrocknet. Das getrocknete Produkt wird grob zerstoßen, gemahlen und die Partikel der Größe 150 - 850 µm zur weiteren Umsetzung ausgesiebt (Pulver D).

50 g Pulver D wird unter kräftigem Rühren mit einer Lösung 0,5 g Ethylencarbonat und 1,5 g Wasser vermischt und danach für 60 min. in einem Ofen, der auf 170 °C temperiert ist, erhitzt.

### Beispiel 3

### (NG: 50; 0,012 g Silbernitrat)

In 955,997 g einer wässrigen Lösung von Natriumacrylat mit einem Neutralisationsgrad von 50 Mol-% (Monomer-Konzentration: 37,7 %) werden 0,45 g Polyethylenglycol-300-diacrylat und 1,05 g Polyethylenglycol-750-monoallyletheracrylat als Vernetzer gelöst. 0,012 g Silbernitrat werden in 10 g Wasser gelöst und in die Monomerenlösung gegeben. Anschließend wird die Monomerenlösung in einem Kunststoff-Polymerisationsgefäß für 30 Minuten mit Stickstoff durchspült, um den gelösten Sauerstoff zu entfernen. Bei einer Temperatur von 4 °C wird die Polymerisation durch die aufeinanderfolgende Zugabe von 0,3 g Natrium-peroxidisulfat in 10 g dest. Wasser, 0,1 g 2,2'-Azobis-2-amidinopropandihydrochlorid in 10 g dest. Wasser, 0,07 g 35 %ige Wasserstoffperoxidlösung in 10 g dest. Wasser und 0,015 g Ascorbinsäure in 2 g dest. Wasser gestartet. Nachdem die Endtemperatur (ca. 100 °C) erreicht ist, wird das Gel mit einem Fleischwolf zerkleinert und 2 h bei 150 °C in einem Umluftofen getrocknet. Das getrocknete Produkt wird grob zerstoßen, gemahlen und die Partikel der Größe 150 - 850 µm zur weiteren Umsetzung ausgesiebt (Pulver E).

50 g Pulver E wird unter kräftigem Rühren mit einer Lösung 0,5 g Ethylencarbonat und 1,5 g Wasser vermischt und danach für 40 min. in einem Ofen, der auf 160 °C temperiert ist, erhitzt.

### Vergleichsbeispiel 3

In 965,988 g einer wässrigen Lösung von Natriumacrylat mit einem Neutralisationsgrad von 50 Mol-% (Monomer-Konzentration: 37,7 %) werden 0,45 g Polyethylenglycol-300-diacrylat und 1,05 g Polyethylenglycol-750-monoallyletheracrylat als Vernetzer gelöst. Anschließend wird die Monomerenlösung in einem Kunststoff-Polymerisationsgefäß für 30 Minuten mit Stickstoff durchspült, um den gelösten Sauerstoff zu entfernen. Bei einer Temperatur von 4 °C wird die Polymerisation durch die aufeinanderfolgende Zugabe von 0,3 g Natrium-peroxidisulfat in 10 g dest. Wasser, 0,1 g 2,2'-Azobis-2-amidinopropandihydrochlorid in 10 g dest. Wasser, 0,07 g 35 %ige Wasserstoffperoxidlösung in 10 g dest. Wasser und 0,015 g Ascorbinsäure in 2 g dest. Wasser gestartet. Nachdem die Endtemperatur (ca. 100 °C) erreicht ist, wird das Gel mit einem Fleischwolf zerkleinert und 2 h bei 150 °C in einem Umluftofen getrocknet. Das getrocknete Produkt wird grob zerstoßen, gemahlen und die Partikel der Größe 150 - 850 µm zur weiteren Umsetzung ausgesiebt (Pulver F).

50 g Pulver F wird unter kräftigem Rühren mit einer Lösung 0,5 g Ethylencarbonat und 1,5 g Wasser vermischt und danach für 40 min. in einem Ofen, der auf 160 °C temperiert ist, erhitzt.

**Tabelle**

| Nummer | Messung | Verzögerungs zeitraum * |
|---|---|---|
| 1 | Nullwert (ohne SAP) | 2 h |
| 2 | Bsp. 1 | 14 h |
| 3 | Bsp. 2 | 7,5 h |
| 4 | Vergl. 1 | 5 h |
| 5 | Vergl. 2 | 3 h |
| 6 | Bsp. 3 | 9 h |
| 7 | Vergl. 3 | 5 h |

| | | |
|---|---|---|
| * Verzögerungszeitraum der bakteriellen Zersetzungsaktivität bis zu einer NH₃-Freisetzung von > 200 ppm x h | | |

Aus der Tabelle wird ersichtlich, dass durch den Zusatz geringer Mengen Silbersalz ein deutlicher bakteriostatischer Effekt erzielt wird. Derartige Superabsorber eigenen sich deshalb in hervorragender Weise zur Geruchskontrolle beim Einsatz zum Beispiel in Inkontinenzartikeln. Weiterhin wird deutlich, dass silberfreie Superabsorber die Ammoniakbildung deutlich verzögern können.

Aus Vergleichsbeispiel 1 ist ebenfalls ersichtlich, dass ein weitgehend unlösliches Silbersalz wie Silberchlorid nicht die Effektivität wie Silbernitrat erreicht.

Ferner zeigen die Beispiele 2 und 3, dass selbst bei Silbersalze enthaltenden und erniedrigtem Neutralisationsgrad aufweisenden erfindungsgemäßen wasserabsorbierenden Polymerteilchen ein deutlicher bakteriostatischer Effekt erzielt wird, der es ermöglicht bei sehr niedrigen Silberkonzentrationen eine effektive Retardation einer Ammoniakfreisetzung zu erreichen.

## Patentansprüche

1. Wasserabsorbierendes, antimikrobielles Polymerteilchen beinhaltend
- 1 bis 500 ppm, bezogen auf das Polymerteilchen, eines auf einem Silbersalz basierenden Silberions, wobei das Silbersalz AgₘXₙ ein Löslichkeitsprodukt von mindestens 1 × 10⁻⁸ (Mol/l)^{m+n} besitzt;
- mindestens 10 Gew.-%, bezogen auf das Polymerteilchen, eines wasserabsorbierenden Polymers basierend auf:
(α1) 50 bis 99,99 Gew.-% polymerisierten, ethylenisch ungesättigten, säuregruppenhaltigen Monomeren oder deren Salze oder deren Mischungen,
(α2) 0 bis 40 Gew.-% polymerisierten, monoethylenisch ungesättigten, mit α1 polymerisierbaren Monomeren,
(α3) 0,01 bis 5 Gew.-% eines oder mehrerer Vernetzer, wobei diese Vernetzer Verbindungen sind, die mindestens zwei ethylenisch ungesättigte Gruppen innerhalb eines Moleküls aufweisen (Vernetzerklasse I), Verbindungen, die mindestens zwei funktionelle Gruppen aufweist, die mit funktionellen Gruppen der Monomeren (α1) oder (α2) in einer Kondensationsreaktion, in einer Additionsreaktion oder in einer Ringöffnungsreaktion reagieren können (Vernetzerklasse II) Verbindungen, die mindestens eine ethylenisch ungesättigte Gruppe und mindestens eine funktionelle Gruppe, die mit funktionellen Gruppen der Monomeren (α1) oder (α2) in einer Kondensationsreaktion, in einer Additionsreaktion oder in einer Ringöffnungsreaktion reagieren kann (Vernetzerklasse III), aufweisen, oder polyvalente Metallkationen (Vernetzerklasse IV),
(α4) 0 bis 30 Gew.-% eines wasserlöslichen Polymeren sowie
(α5) 0 bis 20 Gew.-% eines oder mehrerer Hilfsstoffe, wobei als Hilfsstoffe, Stellmittel, Geruchsbinder, oberflächenaktive Mittel oder Antioxidantien eingesetzt werden,
wobei die Summe der Gewichtsmengen (α1) bis (α5) 100 Gew.-% beträgt;
- gegebenenfalls geeignete Zusatzstoffe, die zur Stabilisierung des Silberions oder des Silbersalzes beitragen und Stoffe die durch Silber bedingte Verfärbung unterbinden oder zumindest aufhalten;
wobei die Konzentration des auf einem Silbersalz basierendes Silberions in höchstens 90 Vol.-% des wasserabsorbierenden, antimikrobiellen Polymerteilchens kleiner als 0,01 ppm ist und wobei
X in der Formel Silbersalz AgₘXₙ für ein Anion aus der Gruppe von Nitrat, Carbonat, Sulfat, Hydrogensulfat, Alaun, Phosphat, Acrylat, Lactat, Acetat, Sulfonat, Benzoat, Triflourmethansulfonat oder Acrylat steht und wobei das wasserabsorbierende, antimikrobielle Polymerteilchen mit einem Nachvernetzer, in einer Menge in einem Bereich von 0,01 bis 30 Gew.-%, bezogen auf das Gewicht des Polymerteilchens, nachvernetzt ist.

2. Wasserabsorbierendes, antimikrobielles Polymerteilchen nach Anspruch 1, **dadurch gekennzeichnet, dass** als Nachvernetzer bevorzugt 1,3-Dioxolan-2-on (Ethylencarbonat) verwendet wird.

3. Wasserabsorbierendes, antimikrobielles Polymerteilchen nach Anspruch 1 beinhaltend
- 1 bis 500 ppm, bezogen auf das Polymerteilchen, eines auf einem Silbersalz basierenden Silberions, wobei das Silbersalz ein Löslichkeitsprodukt von mindestens 1 × 10⁻⁸ (Mol/l)^{m+n} besitzt;
- mindestens 10 Gew.-%, bezogen auf das Polymerteilchen, eines wasserabsorbierenden Polymers basierend auf:
(α1) 50 bis 99,99 Gew.-% polymerisierten, ethylenisch ungesättigten, säuregruppenhaltigen Monomeren oder deren Salze oder deren Mischungen,
(α2) 0 bis 40 Gew.-% polymerisierten, monoethylenisch ungesättigten, mit α1 polymerisierbaren Monomeren,
(α3) 0,01 bis 5 Gew.-%, eines oder mehrerer Vernetzer,
(α4) 0 bis 30 Gew.-% eines wasserlöslichen Polymeren sowie (α5) 0 bis 20 Gew.-% eines oder mehrerer Hilfsstoffe,
wobei die Summe der Gewichtsmengen (α1) bis (α5) 100 Gew.-% beträgt;
- gegebenenfalls geeignete Zusatzstoffe; wobei die Konzentration des auf einem Silbersalz basierendes Silberions in keinem Volumenteil des wasserabsorbierenden, antimikrobiellen Polymerteilchens kleiner als 0,01 ppm ist und wobei
das wasserabsorbierende, antimikrobielle Polymerteilchen mit einem Nachvernetzer in einer Menge in einem Bereich von 0,01 bis 30 Gew.-%, bezogen auf das Gewicht des Polymerteilchens, nachvernetzt ist.

4. Wasserabsorbierendes, antimikrobielles Polymerteilchen nach einem der vorhergehenden Ansprüche beinhaltend
- 1 bis 500 ppm, bezogen auf das Polymerteilchen, eines auf einem Silbersalz basierenden Silberions, wobei das Silbersalz ein Löslichkeitsprodukt von mindestens 1 × 10⁻⁸ (Mol/l)^{m+n} besitzt;
- mindestens 10 Gew.-%, bezogen auf das Polymerteilchen, eines wasserabsorbierenden Polymers basierend auf:
(α1) 50 bis 99,99 Gew.-% polymerisierten, ethylenisch ungesättigten, säuregruppenhaltigen Monomeren oder deren Salze oder deren Mischungen,
(α2) 0 bis 40 Gew.-% polymerisierten, monoethylenisch ungesättigten, mit α1 polymerisierbaren Monomeren,
(α3) 0,01 bis 5 Gew.-%, eines oder mehrerer Vernetzer,
(α4) 0 bis 30 Gew.-% eines wasserlöslichen Polymeren sowie (α5) 0 bis 20 Gew.-% eines oder mehrerer Hilfsstoffe,
wobei die Summe der Gewichtsmengen (α1) bis (α5) 100 Gew.-% beträgt;
- gegebenenfalls geeignete Zusatzstoffe;
wobei die Konzentration des auf einem Silbersalz basierendes Silberions in höchstens 90 Vol.-% des wasserabsorbierenden, antimikrobiellen Polymerteilchens kleiner als 0,01 ppm ist und wobei
der pH-Wert des wasserabsorbierenden, antimikrobiellen Polymerteilchen in einem Bereich von 4,5 bis 7 liegt.

5. Wasserabsorbierendes, antimikrobielles Polymerteilchen nach einem der vorhergehenden Ansprüche beinhaltend
- 1 bis 500 ppm, bezogen auf das Polymerteilchen, eines auf einem Silbersalz basierenden Silberions, wobei das Silbersalz ein Löslichkeitsprodukt von mindestens 1 × 10⁻⁸ (Mol/l)^{m+n} besitzt;
- mindestens 10 Gew.-%, bezogen auf das Polymerteilchen, eines wasserabsorbierenden Polymers basierend auf:
(α1) 50 bis 99,99 Gew.-% polymerisierten, ethylenisch ungesättigten, säuregruppenhaltigen Monomeren oder deren Salze oder deren Mischungen,
(α2) 0 bis 40 Gew.-% polymerisierten, monoethylenisch ungesättigten, mit α1 polymerisierbaren Monomeren,
(α3) 0,01 bis 5 Gew.-% eines oder mehrerer Vernetzer,
(α4) 0 bis 30 Gew.-% eines wasserlöslichen Polymeren sowie (α5) 0 bis 20 Gew.-% eines oder mehrerer Hilfsstoffe,
wobei die Summe der Gewichtsmengen (α1) bis (α5) 100 Gew.-% beträgt;
- gegebenenfalls geeignete Zusatzstoffe;
wobei die Konzentration des auf einem Silbersalz basierendes Silberions in keinem Volumenteil des wasserabsorbierenden, antimikrobiellen Polymerteilchens kleiner als 0,01 ppm ist und wobei
der pH-Wert des wasserabsorbierenden, antimikrobiellen Polymerteilchen in einem Bereich von 4,5 bis 7 liegt.

6. Wasserabsorbierendes, antimikrobielles Polymerteilchen nach einer der vorhergehenden Ansprüche **dadurch gekennzeichnet, dass** (α 3) 0,1 bis 3 Gew.% eines oder mehrerer Vernetzer verwendet werden.

7. Verfahren zur Herstellung eines wasserabsorbierenden, antimikrobiellen Polymerteilchens, wobei als Edukte
(β1) 55 bis 99,99 Gew.-% polymerisierten, ethylenisch ungesättigten, säuregruppenhaltigen Monomeren oder deren Salze oder deren Mischungen,
(β2) 0 bis 40 Gew.-% polymerisierten, monoethylenisch ungesättigten, mit (β1) copolymerisierbaren Monomeren,
(β3) 0,01 bis 5 Gew.-% eines oder mehrerer Vernetzer, wobei diese Vernetzer Verbindungen sind, die mindestens zwei ethylenisch ungesättigte Gruppen innerhalb eines Moleküls aufweisen (Vernetzerklasse I), Verbindungen, die mindestens zwei funktionelle Gruppen aufweisen, die mit funktionellen Gruppen der Monomeren (α1) oder (α2) in einer Kondensationsreaktion, in einer Additionsreaktion oder in einer Ringöffnungsreaktion reagieren können (Vernetzerklasse II) Verbindungen, die mindestens eine ethylenisch ungesättigte Gruppe und mindestens eine funktionelle Gruppe, die mit funktionellen Gruppen der Monomeren (α1) oder (α2) in einer Kondensationsreaktion, in einer Additionsreaktion oder in einer Ringöffnungsreaktion reagieren kann (Vernetzerklasse III), aufweisen, oder polyvalente Metallkationen (Vernetzerklasse IV),
(β4) 0 bis 30 Gew.-% eines wasserlöslichen Polymeren, sowie
(β5) 0 bis 20 Gew.-% eines oder mehrerer Hilfsmittel, wobei als Hilfsstoffe, Stellmittel, Geruchsbinder, oberflächenaktive Mittel oder Antioxidantien eingesetzt werden,
wobei die Summe der Gewichtsmengen (β1) bis (β5) 100 Gew.-% beträgt, miteinander unter Bildung eines wasserabsorbierenden Polymers polymerisiert werden;
wobei 1 bis 500 ppm eines Silberions in Form eines wasserlöslichen Silbersalzes AgₘXₙ mit einem Löslichkeitsprodukt von mindestens 1 × 10⁻⁸ (Mol/l)^{m+n} in einem Lösemittel gelöst, bezogen auf die Edukte, vor Abschluss der Bildung des wasserabsorbierenden Polymers den Edukten zugesetzt wird, und wobei X in der Formel Silbersalz AgₘXₙ für ein Anion aus der Gruppe von Nitrat, Carbonat, Sulfat, Hydrogensulfat, Alaun, Phosphat, Acrylat, Lactat, Acetat, Sulfonat, Benzoat, Triflourmethansulfonat oder Acrylat steht,
und wobei das wasserabsorbierende Polymer zerkleinert, getrocknet und gegebenenfalls gemahlen wird und in einem Nachvernetzungsschritt mit einem Nachvernetzer, ausgewählt aus der Gruppe von Diethylenglykol, Triethylenglykol, Polyethylenglykol, Glyzerin, Polyglyzerin, Propylenglykol, Diethanolamin, Triethanolamin, Polyoxypropylen, Oxyethylen-Oxypropylen-Blockcopolymere, Sorbitanfettsäureester, Polyoxyethylen-sorbitanfettsäureester, Trimethylolpropan, Pentaerytrit, Polyvinylalkohol, Sorbitol, 1,3-Dioxolan-2-on (Ethylencarbonat), 4-Methyl-1,3-dioxolan-2-on (Propylencarbonat), 4,5-Dimethyl-1,3-dioxolan-2-on, 4,4-Dimethyl-1,3-dioxolan-2-on, 4-Ethyl-1,3-dioxolan-2-on, 4-Hydroxymethyl-1,3-dioxolan-2-on, 1,3-Dioxan-2-on, 4-Methyl-1,3-dioxan-2-on, 4,6-Dimethyl-1,3-dioxan-2-on, 1,3-Dioxolan-2-on, Poly-1,3-dioxolan-2-on, in einer Menge in einem Bereich von 0,01 bis 30 Gew.-%, bezogen auf das noch unbehandelte Polymer, nachvernetzt wird.

8. Verfahren zur Herstellung eines wasserabsorbierenden, antimikrobiellen Polymerteilchens nach Anspruch 6, wobei als Edukte
(β1) 55 bis 99,99 Gew.-% polymerisierten, ethylenisch ungesättigten, säuregruppenhaltigen Monomeren oder deren Salze oder deren Mischungen,
(β2) 0 bis 40 Gew.-% polymerisierten, monoethylenisch ungesättigten, mit (β1) copolymerisierbaren Monomeren,
(β3) 0,01 bis 5 Gew.-% eines oder mehrerer Vernetzer,
(β4) 0 bis 30 Gew.-% eines wasserlöslichen Polymeren, sowie (β5) 0 bis 20 Gew.-% eines oder mehrerer Hilfsmittel,
wobei die Summe der Gewichtsmengen (β1) bis (β5) 100 Gew.-% beträgt, miteinander unter Bildung eines wasserabsorbierenden Polymers polymerisiert werden;
wobei 1 bis 500 ppm eines Silberions in Form eines wasserlöslichen Silbersalzes mit einem Löslichkeitsprodukt von mindestens 1 × 10⁻⁸ (Mol/l)^{m+n} in einem Lösemittel gelöst, bezogen auf die Edukte, vor Abschluss der Bildung des wasserabsorbierenden Polymers den Edukten zugesetzt wird,
und wobei die ethylenisch ungesättigten, säuregruppenhaltigen Monomere (β1) dergestalt neutralisiert werden, das der pH-Wert der wasserabsorbierenden antimikrobiellen Polymerteilchen im Bereich von 4,5 bis 7 liegt.

9. Verfahren zur Herstellung eines wasserabsorbierenden, antimikrobiellen Polymerteilchens nach einem der Ansprüche 7 oder 8, wobei als Edukte (β 3) 0,1 bis 3 Gew.-% eines oder mehrerer Vernetzer verwendet werden.

10. Wasserabsorbierende, antimikrobielle Polymerteilchen erhältlich nach einem Verfahren nach einem der Ansprüche 7 bis 9.

11. Polymerteilchen und Verfahren nach einem der vorhergehenden Ansprüche, wobei 50 bis 80 Mol-% der Säuregruppen aus den ethylenisch ungesättigten, säuregruppenhaltigen Monomeren des wasserabsorbierenden Polymers mit einem Alkali- oder Erdalkalisalz neutralisiert sind.

12. Polymerteilchen und Verfahren nach einem der vorhergehenden Ansprüche, wobei das Silbersalz Sibernitrat ist.

13. Polymerteilchen nach einem der Ansprüche 1 bis 6 und 10 bis 12 mit mindestens einer der nachfolgenden Eigenschaften:
(A) die maximale Aufnahme von 0,9 Gew.-%er NaCl-Lösung liegt nach ERT 440.1-99 liegt in einem Bereich von mindestens 10 bis 1000 g/g;
(B) der mit 0,9 Gew.-%er wässriger NaCl-Lösung extrahierbare Anteil beträgt nach ERT 470.1-99 weniger als 30 Gew.-%, jeweils bezogen auf das Polymer;
(C) die Schüttdichte liegt nach ERT 460.1-99 im Bereich von 300 bis 1000 g/l;
(D) der pH-Wert von 1 g des Polymeren in 1 I Wasser liegt gemäß ERT 400.1-99 im Bereich von 4 bis 10;
(E) die Centrifugation Retention Capacity (CRC) gemäß ERT 441.1-99 liegt im Bereich von 10 bis 100 g/g.

14. Fasern, Folien, Schäume oder Formkörper, beinhaltend ein Polymerteilchen nach einem der Ansprüche 1 bis 6 und 10 bis 12.

15. Verbund, beinhaltend ein Polymerteilchen nach einem der Ansprüche 1 bis 6 und 10 bis 12 sowie ein Substrat.

16. Verwendung eines Polymerteilchen nach einem der Ansprüche 1 bis 6 und 10 bis 12 in Fasern, Folien, Schäumen, Formkörpern oder Verbunden.

17. Verwendung eines Polymerteilchen nach einem der Ansprüche 1 bis 6 und 10 bis 12 oder von Fasern, Folien, Schäumen oder Formkörpern nach Anspruch 14; oder eines Verbunds nach Anspruch 15; oder mindestens zwei davon, nach einem der vorhergehenden Ansprüche zur Herstellung eines Hygieneartikels, zur Vorbeugung oder Behandlung von durch Körperausscheidungen hervorgerufenen Hautirritationen.

## Claims

1. Water-absorbing, antimicrobial polymer particle comprising
- 1 to 500 ppm, based on the polymer particle, of a silver ion based on a silver salt, wherein the silver salt AgₘXₙ has a solubility product of at least 1 x 10⁻⁸ (mol/l)^{m+n};
- at least 10% by weight, based on the polymer particle, of a water-absorbing polymer based on:
(α1) 50 to 99.99% by weight polymerized, ethylenically unsaturated, acid group-containing monomers or salts thereof or mixtures thereof,
(α2) 0 to 40% by weight polymerized, monoethylenically unsaturated monomers polymerizable with α1,
(α3) 0.01 to 5% by weight of one or more cross-linkers, wherein these cross-linkers are compounds having at least two ethylenically unsaturated groups within one molecule (cross-linker class I), compounds having at least two functional groups which can react with functional groups of the monomers (α1) or (α2) in a condensation reaction, in an addition reaction or in a ring-opening reaction (cross-linker class II), compounds having at least one ethylenically unsaturated group and at least one functional group which can react with functional groups of the monomers (α1) or (α2) in a condensation reaction, in an addition reaction or in a ring-opening reaction (cross-linker class III), or polyvalent metal cations (cross-linker class IV),
(α4) 0 to 30% by weight of a water-soluble polymer and also
(α5) 0 to 20% by weight of one or more auxiliaries, wherein the auxiliaries used are modifiers, odour binders, surfactants or antioxidants,
wherein the sum total of amounts by weight of (α1) to (α5) is 100% by weight;
- optionally suitable additives which contribute to the stabilisation of the silver ion or the silver salt and substances which prevent or at least delay discolouration caused by silver;
wherein the concentration of the silver ion based on a silver salt is less than 0.01 ppm in at most 90% by volume of the water-absorbing, antimicrobial polymer particle and where
X in the silver salt formula AgₘXₙ is an anion from the group of nitrate, carbonate, sulphate, hydrogen sulphate, alum,
phosphate, acrylate, lactate, acetate, sulphonate, benzoate, trifluoromethanesulphonate or acrylate and wherein the water-absorbing, antimicrobial polymer particles are postcrosslinked with a postcrosslinker in an amount in a range from 0.01 to 30% by weight, based on the weight of the polymer particle.

2. Water-absorbing, antimicrobial polymer particle according to Claim 1, **characterized in that** the postcrosslinker used is preferably 1,3-dioxolan-2-one (ethylene carbonate).

3. Water-absorbing, antimicrobial polymer particle according to Claim 1 comprising
- -1 to 500 ppm, based on the polymer particle, of a silver ion based on a silver salt, wherein the silver salt has a solubility product of at least 1 x 10⁻⁸ (mol/l)^{m+n} ;
- at least 10% by weight, based on the polymer particle, of a water-absorbing polymer based on:
(α1) 50 to 99.99% by weight polymerized, ethylenically unsaturated, acid group-containing monomers or salts thereof or mixtures thereof,
(α2) 0 to 40% by weight polymerized, monoethylenically unsaturated monomers polymerizable with α1,
(α3) 0.01 to 5% by weight of one or more cross-linkers,
(α4) 0 to 30% by weight of a water-soluble polymer and also
(α5) 0 to 20% by weight of one or more auxiliaries,
wherein the sum total of amounts by weight of (α1) to (α5) is 100% by weight;
- optionally suitable additives;
wherein the concentration of the silver ion based on a silver salt is not less than 0.01 ppm in any part by volume of the water-absorbing, antimicrobial polymer particle and wherein
the water-absorbing, antimicrobial polymer particle is postcrosslinked with a postcrosslinker in an amount in a range from 0.01 to 30% by weight, based on the weight of the polymer particle.

4. Water-absorbing, antimicrobial polymer particle according to any of the preceding claims comprising
- 1 to 500 ppm, based on the polymer particle, of a silver ion based on a silver salt, wherein the silver salt has a solubility product of at least 1 x10⁻⁸ (mol/l)^{m+n};
- at least 10% by weight, based on the polymer particle, of a water-absorbing polymer based on:
(α1) 50 to 99.99% by weight polymerized, ethylenically unsaturated, acid group-containing monomers or salts thereof or mixtures thereof,
(α2) 0 to 40% by weight polymerized, monoethylenically unsaturated monomers polymerizable with α1,
(α3) 0.01 to 5% by weight of one or more cross-linkers,
(α4) 0 to 30% by weight of a water-soluble polymer and also
(α5) 0 to 20% by weight of one or more auxiliaries,
wherein the sum total of amounts by weight of (α1) to (α5) is 100% by weight;
- optionally suitable additives;
wherein the concentration of the silver ion based on a silver salt is less than 0.01 ppm in at most 90% by volume of the water-absorbing, antimicrobial polymer particle and wherein
the pH of the water-absorbing, antimicrobial polymer particle is in a range from 4.5 to 7.

5. Water-absorbing, antimicrobial polymer particle according to any of the preceding claims comprising
- 1 to 500 ppm, based on the polymer particle, of a silver ion based on a silver salt, wherein the silver salt has a solubility product of at least 1 x10⁻⁸ (mol/l)^{m+n};
- at least 10% by weight, based on the polymer particle, of a water-absorbing polymer based on:
(α1) 50 to 99.99% by weight polymerized, ethylenically unsaturated, acid group-containing monomers or salts thereof or mixtures thereof,
(α2) 0 to 40% by weight polymerized, monoethylenically unsaturated monomers polymerizable with α1,
(α3) 0.01 to 5% by weight of one or more cross-linkers,
(α4) 0 to 30% by weight of a water-soluble polymer and also
(α5) 0 to 20% by weight of one or more auxiliaries,
wherein the sum total of amounts by weight of (α1) to (α5) is 100% by weight;
- optionally suitable additives;
wherein the concentration of the silver ion based on a silver salt is not less than 0.01 ppm in any part by volume of the water-absorbing, antimicrobial polymer particle and wherein
the pH of the water-absorbing, antimicrobial polymer particle is in a range from 4.5 to 7.

6. Water-absorbing, antimicrobial polymer particle according to any of the preceding claims, **characterized in that** (α3) 0.1 to 3% by weight of one or more cross-linkers are used.

7. Method for preparing a water-absorbing, antimicrobial polymer particle, wherein the reactants polymerized with one another to form a water-absorbing polymer are
(β1) 55 to 99.99% by weight polymerized, ethylenically unsaturated, acid group-containing monomers or salts thereof or mixtures thereof,
(β2) 0 to 40% by weight polymerized, monoethylenically unsaturated monomers polymerizable with (β1),
(β3) 0.01 to 5% by weight of one or more cross-linkers, wherein these crosslinkers are compounds having at least two ethylenically unsaturated groups within one molecule (cross-linker class I), compounds having at least two functional groups which can react with functional groups of the monomers (α1) or (α2) in a condensation reaction, in an addition reaction or in a ring-opening reaction (cross-linker class II), compounds having at least one ethylenically unsaturated group and at least one functional group which can react with functional groups of the monomers (α1) or (α2) in a condensation reaction, in an addition reaction or in a ring-opening reaction (cross-linker class III), or polyvalent metal cations (cross-linker class IV),
(β4) 0 to 30% by weight of a water-soluble polymer and also
(β5) 0 to 20% by weight of one or more auxiliaries, wherein the auxiliaries used are modifiers, odour binders, surfactants or antioxidants,
wherein the sum total of amounts by weight of (β1) to (β5) is 100% by weight,
wherein 1 to 500 ppm, based on the reactants,
of a silver ion in the form of a water-soluble silver salt AgₘXₙ having a solubility product of at least 1 x 10⁻⁸ (mol/l)^{m+n}, dissolved in a solvent, is added to the reactants before completion of the formation of the water-absorbing polymer, and where X in the silver salt formula AgₘXₙ is an anion from the group of nitrate, carbonate, sulphate, hydrogen sulphate, alum, phosphate, acrylate, lactate, acetate, sulphonate, benzoate, triflouromethanesulphonate or acrylate,
and wherein the water-absorbing polymer is comminuted, dried and optionally ground and in a postcrosslinking step is postcrosslinked with a postcrosslinker, selected from the group of diethylene glycol, triethylene glycol, polyethylene glycol, glycerol, polyglycerol, propylene glycol, diethanolamine, triethanolamine, polyoxypropylene, oxyethyleneoxypropylene block copolymers, sorbitan fatty acid ester, polyoxyethylene-sorbitan fatty acid ester, trimethylolpropane, pentaerythritol, polyvinyl alcohol, sorbitol, 1,3-dioxolan-2-one (ethylene carbonate), 4-methyl-1,3-dioxolan-2-one (propylene carbonate), 4,5-dimethyl-1,3-dioxolan-2-one, 4,4-dimethyl-1,3-dioxolan-2-one, 4-ethyl-1,3-dioxolan-2-one, 4-hydroxymethyl-1,3-dioxolan-2-one, 1,3-dioxan-2-one, 4-methyl-1,3-dioxan-2-one, 4,6-dimethyl-1,3-dioxan-2-one, 1,3-dioxolan-2-one, poly-1,3-dioxolan-2-one, in an amount in a range of 0.01 to 30% by weight, based on the still untreated polymer.

8. Method for preparing a water-absorbing, antimicrobial polymer particle according to Claim 6, wherein the reactants polymerized with one another to form a water-absorbing polymer are
(β1) 55 to 99.99% by weight polymerized, ethylenically unsaturated,acid group-containing monomers or salts thereof or mixtures thereof,
(β2) 0 to 40% by weight polymerized, monoethylenically unsaturated monomers polymerizable with (β1),
(β3) 0.01 to 5% by weight of one or more cross-linkers,
(β4) 0 to 30% by weight of a water-soluble polymer, and also
(β5) 0 to 20% by weight of one or more auxiliaries,
wherein the sum total of amounts by weight of (β1) to (β5) is 100% by weight; wherein 1 to 500 ppm, based on the reactants, of a silver ion in the form of a water-soluble silver salt having a solubility product of at least 1 x 10⁻⁸ (mol/l)^{m+n} dissolved in a solvent, is added to the reactants before the completion of the formation of the water-absorbing polymer,
and wherein the ethylenically unsaturated, acid group-containing monomers (β1) are neutralized in such a way that the pH of the water-absorbing antimicrobial polymer particle is in the range of 4.5 to 7.

9. Method for preparing a water-absorbing, antimicrobial polymer particle according to either of Claims 7 and 8, wherein 0.1 to 3% by weight of one or more crosslinkers are used as reactant (β3).

10. Water-absorbing, antimicrobial polymer particle obtainable by a method according to any of Claims 7 to 9.

11. Polymer particle and method according to any of the preceding claims, wherein 50 to 80 mol% of the acid groups of the ethylenically unsaturated, acid group-containing monomers of the water-absorbing polymer are neutralized with an alkali metal or alkaline earth metal salt.

12. Polymer particle and method according to any of the preceding claims, wherein the silver salt is silver nitrate.

13. Polymer particle according to any of Claims 1 to 6 and 10 to 12 having at least one of the following properties:
**(A)** the maximum absorption of 0.9% by weight NaCl solution, according to ERT 440.1-99, is in a range of at least 10 to 1000 g/g;
**(B)** the proportion extractable with 0.9% by weight aqueous NaCl solution, according to ERT 470.1-99, is less than 30% by weight, in each case based on the polymer;
**(C)** the bulk density, according to ERT 460.1-99, is in the range of 300 to 1000 g/l;
**(D)** the pH of 1 g of the polymer in 1 1 of water, according to ERT 400.1-99, is in the range of 4 to 10;
**(E)** the centrifugation retention capacity (CRC), according to ERT 441.1-99, is in the range of 10 to 100 g/g.

14. Fibres, films, foams or shaped bodies comprising a polymer particle according to any of Claims 1 to 6 and 10 to 12.

15. Composite comprising a polymer particle according to any of Claims 1 to 6 and 10 to 12 and also a substrate.

16. Use of a polymer particle according to any of Claims 1 to 6 and 10 to 12 in fibres, films, foams or composites.

17. Use of a polymer particle according to any of Claims 1 to 6 and 10 to 12 or of fibres, films, foams or shaped bodies according to Claim 14; or of a composite according to Claim 15; or at least two thereof, according to any of the preceding claims for preparing a hygiene article for the prevention or treatment of skin irritation caused by bodily secretions.

## Revendications

1. Particule polymère antimicrobienne, absorbant l'eau, comprenant
- 1 à 500 ppm, par rapport à la particule polymère, d'un ion d'argent basé sur un sel d'argent, le sel d'argent AgₘXₙ présentant un produit de solubilité d'au moins 1 x 10⁻⁸ (mole/l)^{m+n} ;
- au moins 10% en poids, par rapport à la particule polymère, d'un polymère absorbant l'eau à base de :
(α1) 50 à 99,99% en poids de monomères polymérisés, éthyléniquement insaturés, contenant des groupes acides, ou leurs sels ou leurs mélanges,
(α2) 0 à 40% en poids de monomères polymérisés, éthyléniquement monoinsaturés, polymérisables avec α1,
(α3) 0,01 à 5% en poids d'un ou de plusieurs réticulants, ces réticulants étant des composés qui présentent au moins deux groupes éthyléniquement insaturés dans une molécule (classe de réticulants I), des composés qui présentent au moins deux groupes fonctionnels qui peuvent réagir avec les groupes fonctionnels des monomères (α1) ou (α2) dans une réaction de condensation, dans une réaction d'addition ou dans une réaction avec ouverture de cycle (classe de réticulants II), des composés qui présentent au moins un groupe éthyléniquement insaturé et au moins un groupe fonctionnel qui peut réagir avec les groupes fonctionnels des monomères (α1) ou (α2) dans une réaction de condensation, dans une réaction d'addition ou dans une réaction avec ouverture de cycle (classe de réticulants III), ou des cations métalliques polyvalents (classe de réticulants IV),
(α4) 0 à 30% en poids d'un polymère soluble dans l'eau, ainsi que
(α5) 0 à 20% en poids d'un ou de plusieurs adjuvants, en utilisant comme adjuvants des agents de réglage, des agents liant les odeurs, des agents tensioactifs ou des antioxydants,
la somme des quantités pondérales (α1) à (α5) étant égale à 100% en poids ;
- le cas échéant des additifs appropriés, qui contribuent à la stabilisation de l'ion d'argent ou du sel d'argent et des substances qui suppriment ou du moins qui arrêtent la coloration provoquée par l'argent ;
la concentration de l'ion d'argent basé sur un sel d'argent dans au plus 90% en volume de la particule polymère antimicrobienne, absorbant l'eau étant inférieure à 0,01 ppm et
X dans la formule du sel d'argent AgₘXₙ représentant un anion du groupe formé par nitrate, carbonate, sulfate, hydrogénosulfate, alun, phosphate, acrylate, lactate, acétate, sulfonate, benzoate, trifluorométhanesulfonate ou acrylate et la particule polymère antimicrobienne, absorbant l'eau étant postréticulée avec un postréticulant en une quantité dans la plage de 0,01 à 30% en poids, par rapport au poids de la particule polymère.

2. Particule polymère antimicrobienne, absorbant l'eau, selon la revendication 1, **caractérisée en ce qu'**on utilise de préférence, comme postréticulant, de la 1,3-dioxolan-2-one (éthylènecarbonate).

3. Particule polymère antimicrobienne, absorbant l'eau, selon la revendication 1, comprenant
- 1 à 500 ppm, par rapport à la particule polymère, d'un ion d'argent basé sur un sel d'argent, le sel d'argent présentant un produit de solubilité d'au moins 1 x 10⁻⁸ (mole/l)^{m+n} ;
- au moins 10% en poids, par rapport à la particule polymère, d'un polymère absorbant l'eau à base de :
(α1) 50 à 99,99% en poids de monomères polymérisés, éthyléniquement insaturés, contenant des groupes acides, ou leurs sels ou leurs mélanges,
(α2) 0 à 40% en poids de monomères polymérisés, éthyléniquement monoinsaturés, polymérisables avec α1,
(α3) 0,01 à 5% en poids d'un ou de plusieurs réticulants,
(α4) 0 à 30% en poids d'un polymère soluble dans l'eau,
(α5) 0 à 20% en poids d'un ou de plusieurs adjuvants,
la somme des quantités pondérales (α1) à (α5) étant égale à 100% en poids ;
- le cas échéant des additifs appropriés ;
la concentration de l'ion d'argent basé sur un sel d'argent dans aucune partie volumique de la particule polymère antimicrobienne, absorbant l'eau n'étant inférieure à 0,01 ppm et
la particule polymère antimicrobienne, absorbant l'eau étant postréticulée avec un postréticulant en une quantité dans la plage de 0,01 à 30% en poids, par rapport au poids de la particule polymère.

4. Particule polymère antimicrobienne, absorbant l'eau, selon l'une quelconque des revendications précédentes, comprenant
- 1 à 500 ppm, par rapport à la particule polymère, d'un ion d'argent basé sur un sel d'argent, le sel d'argent présentant un produit de solubilité d'au moins 1 x 10⁻⁸ (mole/l)^{m+n} ;
- au moins 10% en poids, par rapport à la particule polymère, d'un polymère absorbant l'eau à base de :
(α1) 50 à 99,99% en poids de monomères polymérisés, éthyléniquement insaturés, contenant des groupes acides, ou leurs sels ou leurs mélanges,
(α2) 0 à 40% en poids de monomères polymérisés, éthyléniquement monoinsaturés, polymérisables avec α1,
(α3) 0,01 à 5% en poids d'un ou de plusieurs réticulants,
(α4) 0 à 30% en poids d'un polymère soluble dans l'eau,
(α5) 0 à 20% en poids d'un ou de plusieurs adjuvants,
la somme des quantités pondérales (α1) à (α5) étant égale à 100% en poids ;
- le cas échéant des additifs appropriés ;
la concentration de l'ion d'argent basé sur un sel d'argent dans au plus 90% en volume de la particule polymère antimicrobienne, absorbant l'eau étant inférieure à 0,01 ppm et le pH de la particule polymère antimicrobienne, absorbant l'eau, étant situé dans une plage de 4,5 à 7.

5. Particule polymère antimicrobienne, absorbant l'eau, selon l'une quelconque des revendications précédentes, comprenant
- 1 à 500 ppm, par rapport à la particule polymère, d'un ion d'argent basé sur un sel d'argent, le sel d'argent présentant un produit de solubilité d'au moins 1 x 10⁻⁸ (mole/l)^{m+n} ;
- au moins 10% en poids, par rapport à la particule polymère, d'un polymère absorbant l'eau à base de :
(α1) 50 à 99,99% en poids de monomères polymérisés, éthyléniquement insaturés, contenant des groupes acides, ou leurs sels ou leurs mélanges,
(α2) 0 à 40% en poids de monomères polymérisés, éthyléniquement monoinsaturés, polymérisables avec α1,
(α3) 0,01 à 5% en poids d'un ou de plusieurs réticulants,
(α4) 0 à 30% en poids d'un polymère soluble dans l'eau,
(α5) 0 à 20% en poids d'un ou de plusieurs adjuvants,
la somme des quantités pondérales (α1) à (α5) étant égale à 100% en poids ;
- le cas échéant des additifs appropriés ;
la concentration de l'ion d'argent basé sur un sel d'argent dans aucune partie volumique de la particule polymère antimicrobienne, absorbant l'eau n'étant inférieure à 0,01 ppm et le pH de la particule polymère antimicrobienne, absorbant l'eau, étant situé dans une plage de 4,5 à 7.

6. Particule polymère antimicrobienne, absorbant l'eau, selon l'une quelconque des revendications précédentes, **caractérisée en ce qu'**on utilise (α3) 0,1 à 3% en poids d'un ou de plusieurs réticulants.

7. Procédé pour la préparation d'une particule polymère antimicrobienne, absorbant l'eau, dans lequel on polymérise les uns avec les autres avec formation d'un polymère absorbant l'eau, comme produits de départ,
(ß1) 55 à 99,99% en poids de monomères polymérisés, éthyléniquement insaturés, contenant des groupes acides, ou leurs sels ou leurs mélanges,
(ß2) 0 à 40% en poids de monomères polymérisés, éthyléniquement monoinsaturés, copolymérisables avec (ß1),
(ß3) 0,01 à 5% en poids d'un ou de plusieurs réticulants, ces réticulants étant des composés qui présentent au moins deux groupes éthyléniquement insaturés dans une molécule (classe de réticulants I), des composés qui présentent au moins deux groupes fonctionnels qui peuvent réagir avec les groupes fonctionnels des monomères (α1) ou (α2) dans une réaction de condensation, dans une réaction d'addition ou dans une réaction avec ouverture de cycle (classe de réticulants II), des composés qui présentent au moins un groupe éthyléniquement insaturé et au moins un groupe fonctionnel qui peut réagir avec les groupes fonctionnels des monomères (α1) ou (α2) dans une réaction de condensation, dans une réaction d'addition ou dans une réaction avec ouverture de cycle (classe de réticulants III), ou des cations métalliques polyvalents (classe de réticulants IV),
(ß4) 0 à 30% en poids d'un polymère soluble dans l'eau, ainsi que
(ß5) 0 à 20% en poids d'un ou de plusieurs adjuvants, en utilisant comme adjuvants des agents de réglage, des agents liant les odeurs, des agents tensioactifs ou des antioxydants,
la somme des quantités pondérales (ß1) à (ß5) valant 100% en poids ;
1 à 500 ppm, par rapport aux produits de départ, d'un ion d'argent sous forme d'un sel d'argent soluble dans l'eau AgₘXₙ, présentant un produit de solubilité d'au moins 1 x 10⁻⁸ (mole/l)^{m+n}, sous forme dissoute dans un solvant, étant ajoutés aux produits de départ avant la fin de la formation du polymère absorbant l'eau, et
X dans la formule du sel d'argent AgₘXₙ représentant un anion du groupe formé par nitrate, carbonate, sulfate, hydrogénosulfate, alun, phosphate, acrylate, lactate, acétate, sulfonate, benzoate, trifluorométhanesulfonate ou acrylate
et le polymère absorbant l'eau étant réduit en morceaux, séché, le cas échéant broyé et postréticulé, dans une étape de postréticulation, avec un postréticulant, choisi dans le groupe formé par le diéthylèneglycol, le triéthylèneglycol, le polyéthylèneglycol, le glycérol, le polyglycérol, le propylèneglycol, la diéthanolamine, la triéthanolamine, le polyoxypropylène, les copolymères à blocs d'oxyéthylène-oxypropylène, les esters d'acides gras de sorbitane, les esters d'acides gras de polyoxyéthylènesorbitane, le triméthylolpropane, le pentaérythritol, le poly(alcool vinylique), le sorbitol, la 1,3-dioxolan-2-one (éthylènecarbonate), la 4-méthyl-1,3-dioxolan-2-one (propylènecarbonate), la 4,5-diméthyl-1,3-dioxolan-2-one, la 4,4-diméthyl-1,3-dioxolan-2-one, la 4-éthyl-1,3-dioxolan-2-one, la 4-hydroxyméthyl-1,3-dioxolan-2-one, la 1,3-dioxan-2-one, la 4-méthyl-1,3-dioxan-2-one, la 4,6-diméthyl-1,3-dioxan-2-one, la 1,3-dioxolan-2-one, la poly-1,3-dioxolan-2-one, en une quantité dans la plage de 0,01 à 30% en poids, par rapport au polymère non encore traité.

8. Procédé pour la préparation d'une particule polymère antimicrobienne, absorbant l'eau, selon la revendication 6, dans lequel on polymérise les uns avec les autres, avec formation d'un polymère absorbant l'eau, comme produits de départ,
(ß1) 55 à 99,99% en poids de monomères polymérisés, éthyléniquement insaturés, contenant des groupes acides, ou leurs sels ou leurs mélanges,
(ß2) 0 à 40% en poids de monomères polymérisés, éthyléniquement monoinsaturés, copolymérisables avec (ß1),
(ß3) 0,01 à 5% en poids d'un ou de plusieurs réticulants,
(ß4) 0 à 30% en poids d'un polymère soluble dans l'eau, ainsi que
(ß5) 0 à 20% en poids d'un ou de plusieurs adjuvants,
la somme des quantités pondérales (ß1) à (ß5) valant 100% en poids ;
1 à 500 ppm, par rapport aux produits de départ, d'un ion d'argent sous forme d'un sel d'argent soluble dans l'eau, présentant un produit de solubilité d'au moins 1 x 10⁻⁸ (mole/l)^{m+n}, sous forme dissoute dans un solvant, étant ajoutés aux produits de départ avant la fin de la formation du polymère absorbant l'eau,
et les monomères (ß1) éthyléniquement insaturés, contenant des groupes acides étant neutralisés de manière telle que le pH des particules polymères antimicrobiennes, absorbant l'eau se situe dans la plage de 4,5 à 7.

9. Procédé pour la préparation d'une particule polymère antimicrobienne, absorbant l'eau, selon l'une quelconque des revendications 7 ou 8, dans lequel on utilise, comme produits de départ (ß3), 0,1 à 3% en poids d'un ou de plusieurs réticulants.

10. Particule polymère antimicrobienne, absorbant l'eau, pouvant être obtenue selon un procédé selon l'une quelconque des revendications 7 à 9.

11. Particule polymère et procédé selon l'une quelconque des revendications précédentes, 50 à 80% en mole des groupes acides des monomères éthyléniquement insaturés, contenant des groupes acides du polymère absorbant l'eau étant neutralisés avec un sel de métal alcalin ou alcalino-terreux.

12. Particule polymère et procédé selon l'une quelconque des revendications précédentes, le sel d'argent étant du nitrate d'argent.

13. Particule polymère selon l'une quelconque des revendications 1 à 6 et 10 à 12, présentant au moins l'une des propriétés suivantes :
(A) l'absorption maximale d'une solution à 0,9% en poids de NaCl se situe, selon ERT 440.1-99, dans une plage d'au moins 10 à 1000 g/g,
(B) la proportion pouvant être extraite par la solution aqueuse à 0,9% en poids de NaCl est, selon ERT 470.1-99, inférieure à 30% en poids, à chaque fois par rapport au polymère,
(C) la densité apparente se situe, selon ERT 460.1-99, dans la plage de 300 à 1000 g/l ;
(D) le pH de 1 g de polymère dans 1 1 d'eau se situe, selon ERT 400.1-99, dans la plage de 4 à 10 ;
(E) la capacité de rétention par centrifugation (CRC) selon ERT 441.1-99 se situe dans la plage de 10 à 100 g/g.

14. Fibres, feuilles, mousses ou corps moulés, comprenant une particule polymère selon l'une quelconque des revendications 1 à 6 et 10 à 12.

15. Composite, comprenant une particule polymère selon l'une quelconque des revendications 1 à 6 et 10 à 12, ainsi qu'un substrat.

16. Utilisation d'une particule polymère selon l'une quelconque des revendications 1 à 6 et 10 à 12 dans des fibres, des feuilles, des mousses, des corps moulés ou des composites.

17. Utilisation d'une particule polymère selon l'une quelconque des revendications 1 à 6 et 10 à 12 ou de fibres, de feuilles, de mousses ou de corps moulés selon la revendication 14 ; ou d'un composite selon la revendication 15 ; ou d'au moins deux de ceux-ci, selon l'une quelconque des revendications précédentes pour la fabrication d'un article d'hygiène, pour la prévention ou le traitement d'irritations de la peau provoquées par des excrétions corporelles.
